# EUROPÄISCHE PATENTSCHRIFT

(11) **EP 2 934 472 B1**
(45) Veröffentlichungstag und Bekanntmachung des Hinweises auf die Patenterteilung: **13.09.2017**
(21) Anmeldenummer: 13792002.1
(22) Anmeldetag: 15.11.2013
(51) Int. Cl.: A61K 8/49, A61Q 5/10, A61K 8/898, A61Q 5/00

(54) **SCHONENDE OXIDATIVE HAARBEHANDLUNG MIT SILIKON-VORBEHANDLUNG UND BLEICHKRAFTVERSTÄRKER**
GENTLE OXIDATIVE HAIR TREATMENT WITH SILICONE PRE-TREATMENT AND BLEACHING FORCE INTENSIFIER
TRAITEMENT DES CHEVEUX OXYDATIF ET RESPECTUEUX À PRÉTRAITEMENT AU SILICONE ET RENFORÇATEUR DE DÉCOLORATION

(30) Priorität: 19.12.2012 DE 102012223804
(43) Veröffentlichungstag der Anmeldung: 28.10.2015
(73) Patentinhaber: Henkel AG & Co. KGaA, 40589 Düsseldorf (DE)
(72) Erfinder: SCHWEINSBERG, Matthias, 22769 Hamburg (DE); BAEK, Jisook, 20144 Hamburg (DE); KLEEN, Astrid, 20457 Hamburg (DE); SCHULZE ZUR WIESCHE, Erik, DE-22453 Hamburg (DE)
(86) Internationale Anmeldenummer: PCT/EP2013/073940
(87) Internationale Veröffentlichungsnummer: WO 2014/095179

(56) Entgegenhaltungen:
- WO-A2-2011/124402
- WO-A2-2012/079950
- WO-A2-2013/014140
- WO-A2-2013/079299
- US-A- 6 007 801
- US-A- 6 117 420
- US-A1- 2003 121 109
- US-A1- 2008 107 815
- "Kosmetische Zusammensetzungen", IP.COM JOURNAL, IP.COM INC., WEST HENRIETTA, NY, US, 4. August 2011 (2011-08-04), XP013146799, ISSN: 1533-0001

## Beschreibung

Die vorliegende Erfindung betrifft ein schonendes Verfahren zur oxidativen Aufhellung und/oder Färbung keratinischer Fasern, insbesondere von Haaren, bei dem die keratinischen Fasern vor oxidativen Einflüssen geschützt werden.

Beim oxidativen Färben wie auch bei der oxidativen Aufhellung von Haaren tritt das Problem auf, dass es aufgrund der aggressiven Agentien zu Irritationen auf der Kopfhaut und Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die wasserabweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind so genannte Vorbehandlungsmittel auf dem Markt, die das Haar vor dem aggressiven Einfluss schützen sollen. Diese beschweren das Haar aber oft oder beeinträchtigen den Erfolg der im Anschluss stattfindenden Aufhellung bzw. Färbung des Haares; insbesondere die Waschechtheit der Färbung kann durch das Vorbehandlungsmittel verschlechtert sein.

Der vorliegenden Erfindung lag die Aufgabe zugrunde, ein Verfahren zur oxidativen Haarbehandlung mit einer Haar schützenden Vorbehandlung bereitzustellen, das die genannten Nachteile überwindet, ohne den Erfolg einer nachfolgenden oxidativen Haarbehandlung zu konterkarieren. Dabei sollten insbesondere ein Verfahren bereitgestellt werden, bei dem das Haar nicht beschwert wird und auch bei einer nicht unmittelbar vor der oxidativen Haarbehandlung stattfindenden Vorbehandlung die gewünschte Wirkung erzielen, wodurch die Zeitspanne zwischen Vorbehandlung und oxidativer Haarbehandlung verlängert werden kann.

Der Einsatz von aminierten Silikonen in der Haarpflege ist Stand der Technik. Diese werden in Shampoos und insbesondere in Conditionern breit eingesetzt, um dort Pflegeffekte zu entfalten. So offenbart die EP 1771144 B1 haarkonditionierende Mittel mit aminofunktionellen Siliconen. Die dort beschriebenen Mittel sind Nachbehandlungsmittel.

Auch die europäischen Patente EP 1312334 B1 (Aminosilicon und Verdicker) sowie EP 1312335 B1 (Aminosilicon und Conditioner) offenbaren Haarnachbehandlungsmittel.

Es wurde nun gefunden, dass eine Vorbehandlung der keratinischen Fasern mit speziellen 4-Morpholinomethyl-substituierten Silikonen innerhalb eines bestimmten Zeitraums vor einer oxidativen Haarbehandlung zu deutlich verbessertem Haarschutz führt, ohne dass die Ergebnisse der oxidativen Haarbehandlung beeinträchtigt werden. Haarschutz im Sinne der Anmeldung wird insbesondere so verstanden, dass die Struktur der keratinischen Faser, insbesondere des Haars, durch das Oxidationsmittel weniger stark angegriffen wird, so dass die Oberfläche der Faser bzw. des Haars weniger stark aufgeraut wird, die Haarenden weniger stark gesplisst werden, und/oder weniger Haarbruch auftritt. Darüber hinaus können auch besonders gute Färbeergebnisse, insbesondere Färbungen mit einer hohen Waschechtheit, erzielt werden.

Gegenstand der vorliegenden Erfindung ist in einer ersten Ausführungsform Verfahren zur oxidativen Aufhellung und/oder Färbung keratinischer Fasern, insbesondere von Haaren, bei dem
a) ein Vorbehandlungsmittel, das mindestens ein 4-Morpholinomethyl-substituiertes Silikon der Formel (V) enthält, 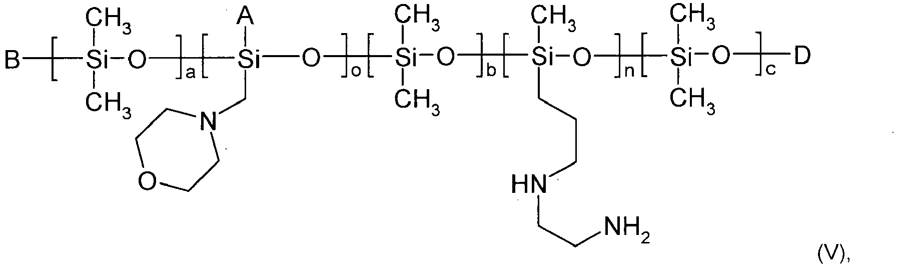 in der
   - A: für eine über ein -O- gebundene Struktureinheit (I), (II), (IIIa), (IIIb), (IIIe) oder (IIIf), 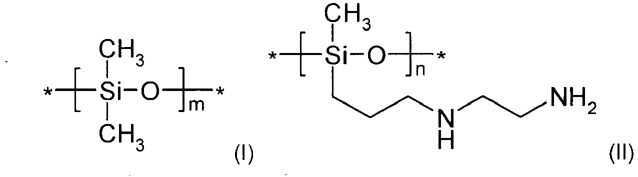 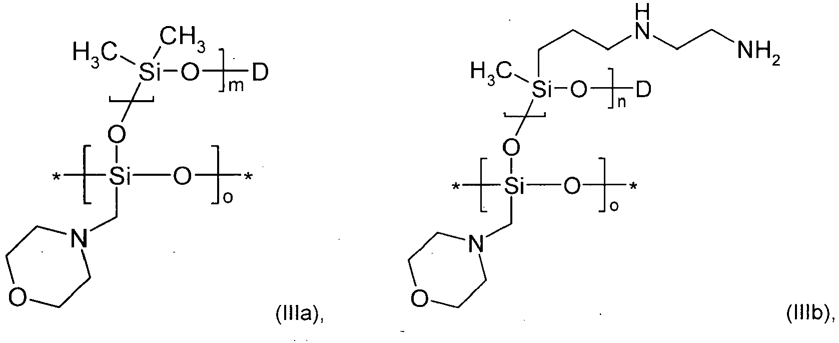 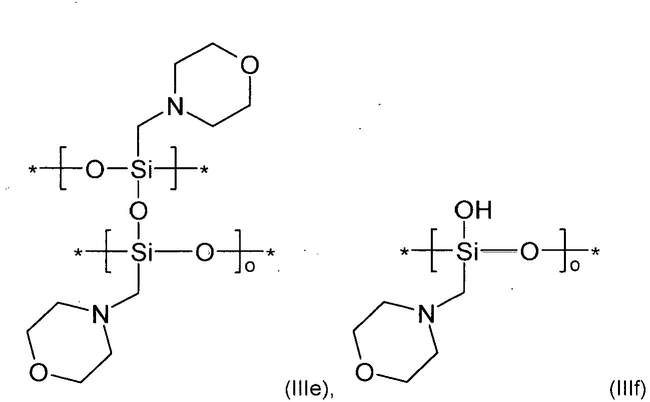 oder für einen über ein -O- gebundenen oligomeren oder polymeren Rest, enthaltend Struktureinheiten der Formeln (I), (II), (IIIa), (IIIb), (IIIe) oder (IIIf) oder für -OH steht,
   - *: für eine Bindung zu einer der Struktureinheiten (I), (II), (IIIa), (IIIb), (IIIe) oder (IIIf) oder für eine Endgruppe B (Si-gebunden) oder D (O-gebunden) steht,
   B für eine Gruppe -OH, -O-Si(CH₃)₃, -O-Si(CH₃)₂OH, -O-Si(CH₃)₂OCH₃ steht,
   D für eine Gruppe -H, -Si(CH₃)₃, -Si(CH₃)₂OH, -Si(CH₃)₂OCH₃ steht,
   a, b und c für ganze Zahlen von 0 bis 990 stehen, mit der Maßgabe a + b + c > 0
   m, n und o für ganze Zahlen von 2 bis 990 stehen,
   auf die keratinischen Fasern, insbesondere die Haare, aufgetragen wird,
b) anschließend innerhalb eines Zeitraumes von einer Sekunde bis 24 Stunden nach Schritt a) ein Färbe- und/oder Aufhellmittel auf die keratinischen Fasern aufgebracht wird, das durch Vermischen einer Zusammensetzung (A), die mindestens ein Alkalisierungsmittel enthält, mit einer Zusammensetzung (B), die in einem kosmetischen Träger mindestens ein Oxidationsmittel enthält, erhalten wird,
   wobei die Zusammensetzung (B) mindestens ein Acylpyridiniumderivat der Formel (Acylpyr-I) 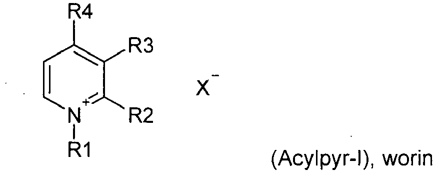
   - R1: für eine C₁-C₆-Alkylgruppe, eine C₂-C₆-Alkenylgruppe, eine C₂-C₆-Hydroxy-alkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
   - R2, R3 und R4: jeweils unabhängig voneinander für Wasserstoff, eine C₁-C₆-Alkylgruppe, ein Halogenatom oder eine C₁-C₆-Acylgruppe steht, mit der Maßgabe, dass zumindest einer der Reste R2, R3 und R4 für eine C₁-C₆-Acylgruppe steht,
   - und X⁻: für ein physiologisch verträgliches Anion steht,
   enthält.

Erfindungsgemäß bevorzugt verwendete Vorbehandlungsmittel sind dadurch gekennzeichnet, dass sie 4-Morpholinomethyl-substituierte(s) Silikon(e) in einer Gesamtmenge von 0,001 bis 5 Gew.-%, bevorzugt 0,005 bis 2 Gew.-%, besonders bevorzugt 0,01 - 1 Gew.-%, außerordentlich bevorzugt 0,02 - 0,1 Gew.-%, jeweils bezogen auf das Gesamtgewicht des Vorbehandlungsmittels, enthalten.

Erfindungsgemäß bevorzugt verwendete Vorbehandlungsmittel sind dadurch gekennzeichnet, dass sie das mindestens eine 4-Morpholinomethyl-substituierte Silikon der Formel (V) in in Wasser emulgierter Form enthalten. Besonders bevorzugt verwendete Vorbehandlungsmittel enthalten 30 - 98 Gew.-%, bevorzugt 40 - 90 Gew.-%, besonders bevorzugt 50 - 85 Gew.-%, außerordentlich bevorzugt 60 - 80 Gew.-% Wasser, jeweils bezogen auf das Gesamtgewicht des Vorbehandlungsmittels.

Besonders bevorzugt verwendete Vorbehandlungsmittel liegen in Form einer Öl-in-Wasser-Emulsion vor, in der die zahlenmittlere Größe der Silicon partikel in der Emulsion im Bereich von 3 bis 500 nm, bevorzugt im Bereich von 5 bis 60 nm liegt.

Die Struktureinheiten der Formeln (I), (II), (IIIa), (IIIb), (IIIe) und (IIIf) können dabei statistisch verteilt im Molekül vorliegen, die erfindungsgemäß eingesetzten Silikone können aber auch Blockcopolymere aus Blöcken der einzelnen Struktureinheiten sein, wobei die Blöcke wiederum statistisch verteilt vorliegen können.

Der * an den freien Valenzen der Struktureinheiten (I), (II), (IIIa), (IIIb), (IIIe) oder (IIIf) steht dabei für eine Bindung zu einer der Struktureinheiten (I), (II), (IIIa), (IIIb), (IIIe) oder (IIIf) oder für eine Endgruppe B (Si-gebunden) oder D (O-gebunden).

Die erfindungsgemäß eingesetzten Silikone können beidseitig Trimethylsilyl-terminiert sein (D = -Si(CH₃)₃, B = -O-Si(CH₃)₃), sie können aber auch ein- oder zweiseitig Dimethylsilylhydroxy- oder Dimethylsilylmethoxy-terminiert sein. Im Rahmen der vorliegenden Erfindung besonders bevorzugt eingesetzte Silikone weisen mindestens eine endständige Dimethylsilylhydroxy-Gruppe auf, d.h. sind ausgewählt aus Silikonen, in denen

| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der nach dem erfindungsgemäßen Verfahren behandelten Haare, insbesondere zu einer gravierenden Verringerung des Kontaktwinkels.

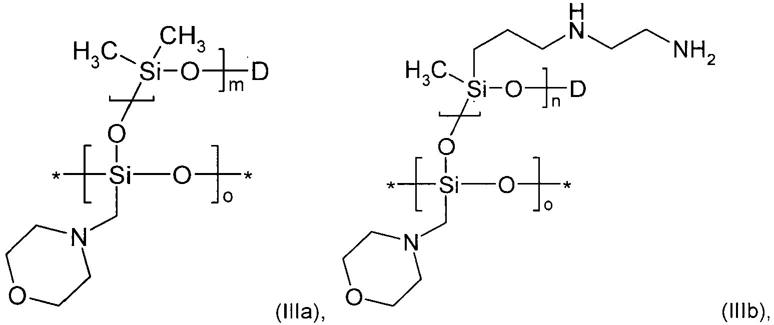

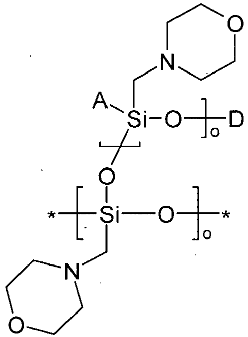

In den vorstehend genannten Formeln (IIIa) und (IIIb) können die Indices m, n und o für ganze Zahlen von 2 bis 990 stehen.

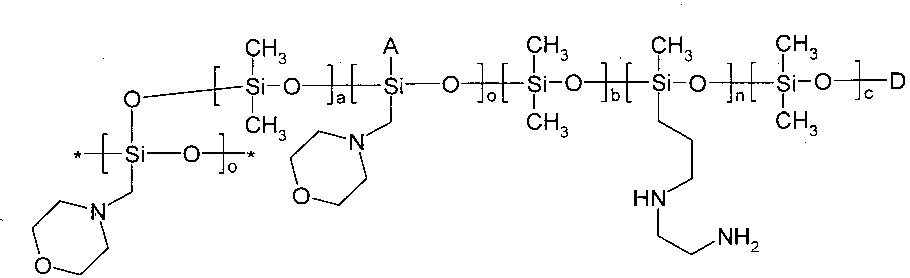

In der Struktureinheit (IIIe) steht A für die Hälfte eines verbindenden O-Atoms zu einer Struktureinheit (III). In der Struktureinheit (IIIf) steht A für -OH.

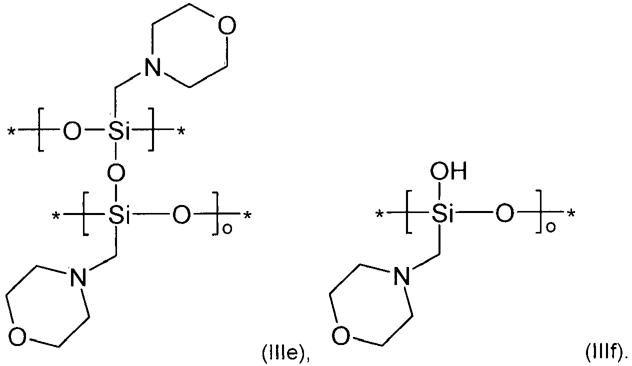

Wie bereits erwähnt, können die Struktureinheiten der Formeln (I), (II), (IIIa), (IIIb), (IIIe) und (IIIf) bevorzugt statistisch verteilt vorliegen.

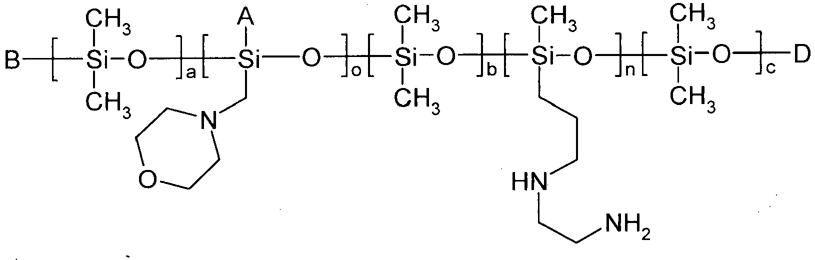

Strukturformel (V) soll verdeutlichen, dass die Siloxangruppen n und o nicht zwingend direkt an eine Endgruppierung B bzw. D gebunden sein müssen. Vielmehr gilt in bevorzugten Formeln (V) a > 0 oder b > 0 und in besonders bevorzugten Formeln (V) a > 0 und b > 0, d.h. die terminale Gruppierung B bzw. D ist bevorzugt an eine Dimethylsiloxy-Gruppierung gebunden. Auch in Formel (V) sind die Siloxaneinheiten a, b, c, n und o bevorzugt statistisch verteilt.

Auch die durch Formel (V) dargestellten erfindungsgemäß eingesetzten Silikone können beidseitig Trimethylsilyl-terminiert sein (D = -Si(CH₃)₃, B = -O-Si(CH₃)₃), sie können aber auch ein- oder zweiseitig Dimethylsilylhydroxy- oder Dimethylsilylmethoxy-terminiert sein. Im Rahmen der vorliegenden Erfindung besonders bevorzugt eingesetzte Silikone weisen mindestens eine endständige Dimethylsilylhydroxy-Gruppe auf, d.h. sind ausgewählt aus Silikonen, in denen

| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese 4-Morpholinomethyl-substituierte Silikon der Formel (V), das jeweils mindestens eine der Struktureinheiten der Formeln (I), (11) (IIIa), (IIIb), (IIIe) und (IIIf) aufweist, führen zu überraschend hohen Verbesserungen der Haareigenschaften der gemäß dem erfindungsgemäßen Verfahren behandelten Haare, insbesondere zu einem gravierend verbesserten Haarschutz und Farbschutz bei der oxidativen Haarfärbung.

Auch in Formel (V) kann der Rest A
- für eine über ein -O- gebundene Struktureinheit (I), (II) (IIIa), (IIIb), (IIIe) oder (IIIf) oder
- einen über ein -O- gebundenen oligomeren oder polymeren Rest enthaltend Struktureinheiten der Formeln (I), (II) (IIIa), (IIIb), (IIIe) oder (IIIf)
stehen.

Analog den Ausführungen zu den Struktureinheiten (IIIa), (IIIb), (IIIe) und (IIIf) wird damit Formel (V) präzisiert zu einer der Formeln (Va), (Vb), (Ve) oder (Vf):

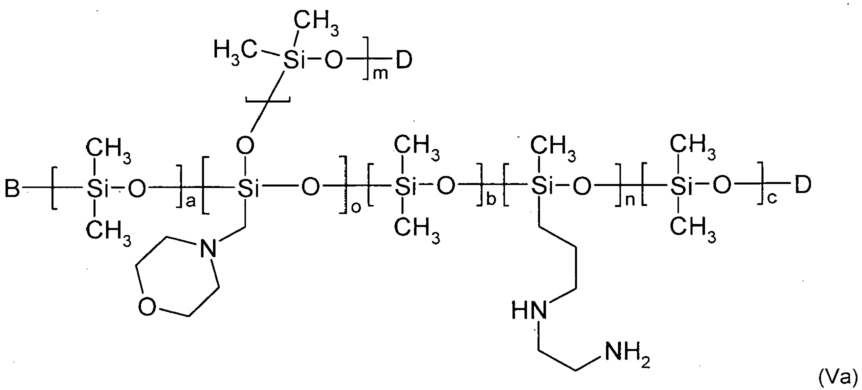

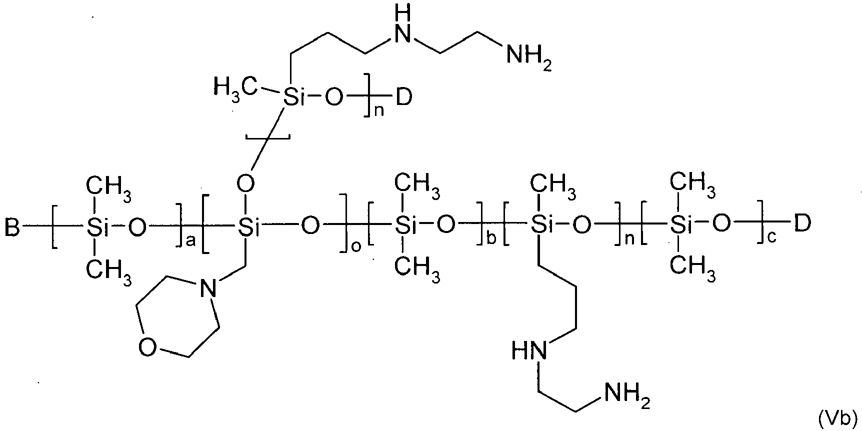

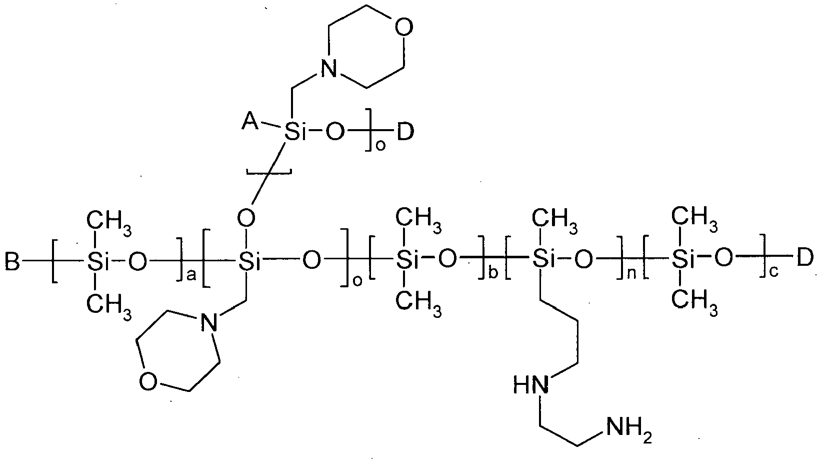

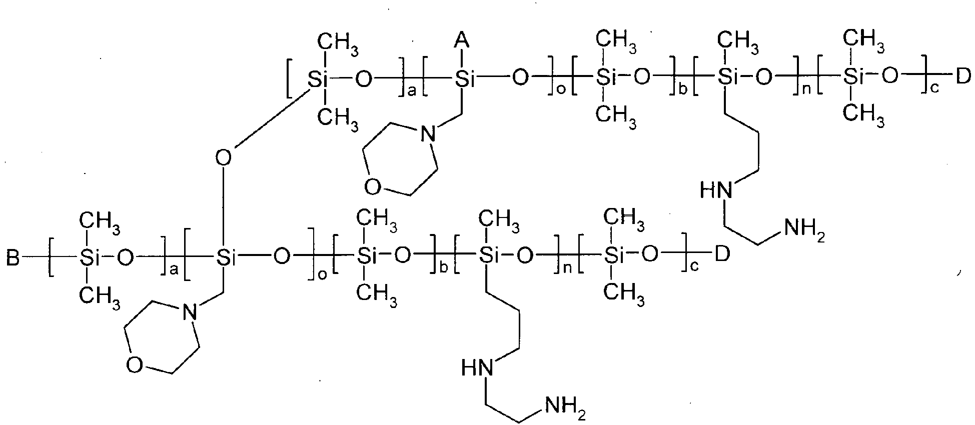

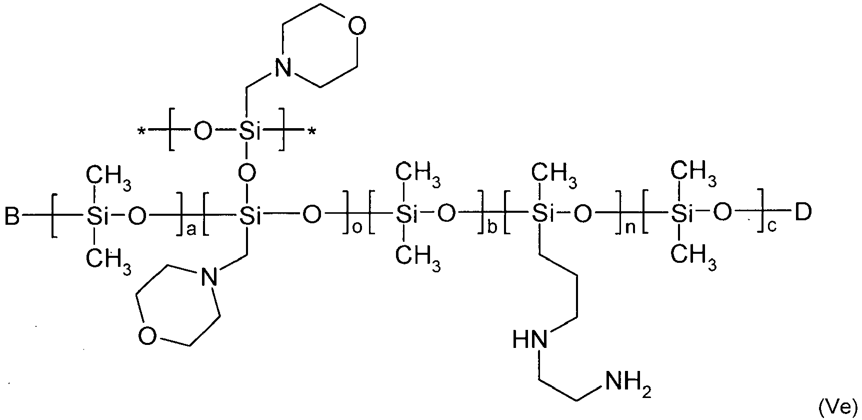

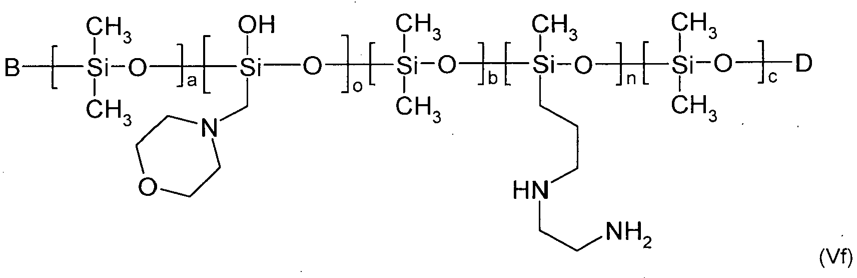

Unabhängig davon, welches spezielle 4-Morpholinomethyl-substituierte Silikon in den erfindungsgemäß verwendeten Vorbehandlungsmitteln enthalten ist, sind für das erfindungsgemäße Verfahren Vorbehandlungsmittel bevorzugt, die ein 4-Morpholinomethyl-substituiertes Silikon enthalten, in dem mehr als 50 Mol-% der Struktureinheiten Dimethylsiloxy-Einheiten sind, d.h. in denen die Struktureinheit (I) mindestens die Hälfte aller Struktureinheiten des eingesetzten Silikons ausmacht.

In anderen Worten sind Silikone bevorzugt, bei denen m > (n + o) bzw. (a+b+c) > (n + o) gilt.

Noch weiter bevorzugte erfindungsgemäß verwendete Vorbehandlungsmittel enthalten ein 4-Morpholinomethyl-substituiertes Silikon, in dem mehr als 87,5 Mol-% der Struktureinheiten Dimethylsiloxy-Einheiten sind, d.h. in denen die Struktureinheit (I) mehr als 875 Tausendstel aller Struktureinheiten des eingesetzten Silikons ausmacht.

In anderen Worten sind Silikone bevorzugt, bei denen m > 8(n + o) bzw. (a+b+c) > 8(n + o) gilt.

Noch weiter bevorzugte erfindungsgemäß verwendete Vorbehandlungsmittel enthalten ein 4-Morpholinomethyl-substituiertes Silikon, in dem mehr als 96 Mol-% der Struktureinheiten Dimethylsiloxy-Einheiten sind, d.h. in denen die Struktureinheit (I) mindestens sechsundneunzig Hundertstel aller Struktureinheiten des eingesetzten Silikons ausmacht.

In anderen Worten sind Silikone bevorzugt, bei denen m > 25(n + o) bzw. (a+b+c) > 25(n + o) gilt.

Noch weiter bevorzugte erfindungsgemäß verwendete Vorbehandlungsmittel enthalten ein 4-Morpholinomethyl-substituiertes Silikon, in dem mehr als 98,7 Mol-% der Struktureinheiten Dimethylsiloxy-Einheiten sind, d.h. in denen die Struktureinheit (I) mindestens neunhundertsiebenundachtzig Tausendstel aller Struktureinheiten des eingesetzten Silikons ausmacht.

In anderen Worten sind Silikone bevorzugt, bei denen m > 77(n + o) bzw. (a+b+c) > 77(n + o) gilt.

Noch weiter bevorzugte erfindungsgemäß verwendete Vorbehandlungsmittel enthalten ein 4-Morpholinomethyl-substituiertes Silikon, in dem mehr als 99,5 Mol-% der Struktureinheiten Dimethylsiloxy-Einheiten sind, d.h. in denen die Struktureinheit (I) mindestens neunhundertfünfundneunzig Tausendstel aller Struktureinheiten des eingesetzten Silikons ausmacht.

In anderen Worten sind Silikone bevorzugt, bei denen m > 200(n + o) bzw. (a+b+c) > 200(n + o) gilt.

Zusammenfassend sind bevorzugte erfindungsgemäß verwendete Vorbehandlungsmittel dadurch gekennzeichnet, dass sie mindestens ein 4-Morpholinomethyl-substituiertes Silikon enthalten, bei dem
- m > (n + o) bzw. (a+b+c) > (n + o), bevorzugt
- m > 8(n + o) bzw. (a+b+c) > 8(n + o), besonders bevorzugt
- m > 25(n + o) bzw. (a+b+c) > 25(n + o), weiter bevorzugt
- m > 77(n + o) bzw. (a+b+c) > 77(n + o) und insbesondere
- m > 200(n + o) bzw. (a+b+c) > 200(n + o) gilt.

Ein weiteres erfindungsgemäß bevorzugtes Verfahren ist dadurch gekennzeichnet, dass das in Schritt a eingesetzte Vorbehandlungsmittel Hydroxy-terminierte(s) 4-Morpholinomethyl-substituierte(s) Silikon(e) enthält, in dem/in denen das Molverhältnis Hydroxy/Alkoxy im Bereich von 0,2:1 bis 0,4:1, bevorzugt im Bereich von 1:0,8 bis 1:1,1 liegt.

Ein weiteres erfindungsgemäß bevorzugtes Verfahren ist dadurch gekennzeichnet, dass die gewichtsmittlere Molmasse des in Schritt a eingesetzten 4-Morpholinomethyl-substituierten Silikons der Formel (V) im Bereich von 2.000 bis 1.000.000 gmol⁻¹, bevorzugt im Bereich von 5.000 bis 200.000 gmol⁻¹ liegt.

Die mittleren Molmassen von aminosubstituierten Silikonen sind beispielsweise durch Gelpermeationschromatographie (GPC) bei Raumtemperatur in Polystyrol messbar. Als Säulen können Styragel Spalten µ, als Eluent THF und als Flussrate 1 ml / min gewählt werden. Die Detektion erfolgt bevorzugt mittels Refraktometrie und UV-Meter.

Erfindungsgemäß besonders bevorzugte 4-Morpholinomethyl-substituierte Silikone der Formel (V) sind in dem Rohstoff Belsil ADM 8301 E (ex Wacker Silicones) unter der Bezeichung Amodimethicone/Morpholinomethyl Silsesquioxane enthalten. Belsil ADM 8301 E stellt eine Mikroemulsion dar und besteht aus folgenden Bestandteilen: Amodimethicone/Morpholinomethyl Silsesquioxane (10 Gew.-%); Trideceth-5 (5 Gew.-%); Glycerin (2,5 Gew.-%), Phenoxyethanol (0,45 Gew.-%) und Wasser (82,05 Gew.-%).

Es hat sich gezeigt, dass das erfindungsgemäße Verfahren weiter verbessert werden kann, wenn bestimmte nichtionische Komponenten ebenfalls in den erfindungsgemäß verwendeten Vorbehandlungsmitteln enthalten sind. Zudem haben diese nichtionischen Komponenten positive Effekte auf die Lagerstabilität der erfindungsgemäß verwendeten Vorbehandlungsmittel. Nichtionische Komponenten, die hier besonders geeignet sind, sind Ethoxylate von Decanol, Undecanol, Dodecanol, Tridecanol, Myristylalkohol, Cetylalkohol und/oder Stearylalkohol. Als besonders geeignet haben sich ethoxylierte Tridecanole erwiesen, die mit besonderem Vorzug in die erfindungsgemäß verwendeten Vorbehandlungsmittel inkorporiert werden. Besonders bevorzugt sind verzweigte ethoxylierte Tridecanole, insbesondere verzweigte Tridecanole mit 3 bis 5 Ethylenoxid-Einheiten im Molekül. Erfindungsgemäß besonders bevorzugt verwendete Vorbehandlungsmittel enthalten - jeweils bezogen auf ihr Gewicht - 0,001 bis 5 Gew.-%, bevorzugt 0,005 - 3,5 Gew.-%, besonders bevorzugt 0,01 - 2 Gew.-%, weiter bevorzugt 0,05 - 1 Gew.-% und insbesondere 0,1 - 0,5 Gew.-% verzweigtes, ethoxyliertes Tridecanol, besonders bevorzugt 0,001 - 5 Gew.-%, bevorzugt 0,005 - 3,5 Gew.-%, besonders bevorzugt 0,01 - 2 Gew.-%, weiter bevorzugt 0,05 - 1 Gew.-% und insbesondere 0,1 - 0,5 Gew.-% verzweigtes, ethoxyliertes Tridecanol mit 3 bis 5 Ethylenoxid-Einheiten im Molekül.

Weitere Tenside und Emulgatoren sind in den erfindungsgemäß verwendeten Vorbehandlungsmitteln bevorzugt nicht oder nur in geringen Mengen enthalten. Erfindungsgemäß bevorzugt verwendete Vorbehandlungsmittel enthalten, bezogen auf das Gesamtgewicht des Mittels, 0,001 bis maximal 6 Gew.-% Tensid(e), wobei die vorgenannten Ethoxylate von Decanol, Undecanol, Dodecanol, Tridecanol, Myristylalkohol, Cetylalkohol und/oder Stearylalkohol mit eingeschlossen sind.

Bevorzugt werden die erfindungsgemäß verwendeten Vorbehandlungsmittel niedrigviskos, das heißt, mit einer Viskosität (bei 20°C gemessen) im Bereich von 10 - 2000 mPas, bevorzugt 20 - 1000 mPas, besonders bevorzugt 50 - 800 mPas, formuliert. Es hat sich darüber hinaus gezeigt, dass verdickende Polymere die erfindungsgemäße Wirkung abschwächen können, so dass bevorzugte erfindungsgemäß verwendete Vorbehandlungsmittel dadurch gekennzeichnet sind, dass sie verdickende Polymere in einer Gesamtmenge von ≤ 2,5 Gew.-%, bevorzugt ≤ 1 Gew.-%, weiter bevorzugt ≤ 0,5 Gew.-% und insbesondere ≤ 0,01 Gew.-%, jeweils bezogen auf das Gewicht des Vorbehandlungsmittels, enthalten.

Die erfindungsgemäß verwendeten Vorbehandlungsmittel können weitere Inhaltsstoffe enthalten. Bevorzugt ist hierbei der Einsatz von mehrwertigen Alkoholen, die Feuchtigkeit spendende Eigenschaften aufweisen. Hier sind erfindungsgemäß verwendete Vorbehandlungsmittel bevorzugt, die mindestens einen mehrwertigen Alkohol, bevorzugt ausgewählt aus der Gruppe Sorbit und/oder Glycerin und/oder 1,2-Propylenglycol oder deren Mischungen, in einer Gesamtmenge von 0,05 - 15 Gew.-%, bevorzugt 0,1 - 10 Gew.-%, besonders bevorzugt 0,15 - 5 Gew.-% und insbesondere 0,15 - 1 Gew.-%, jeweils bezogen auf das Gewicht des Vorbehandlungsmittels, enthalten. Für bestimmte Anwendungsbereiche kann es vorteilhaft sein, nur einen der drei oben genannten bevorzugten mehrwertigen Alkohole einzusetzen. In den meisten Fällen ist dabei Glycerin bevorzugt. Allerdings können auf anderen Anwendungsgebieten Mischungen von zwei der drei mehrwertigen Alkohole oder aller drei mehrwertigen Alkohole bevorzugt sein. Besonders vorteilhaft hat sich hier eine Mischung aus Glycerin, Sorbit und 1,2-Propylenglycol in einem Gewichtsverhältnis von 1 : (0,5-1): (0,1-0,5) erwiesen.

Neben Sorbit, Glycerin und 1,2-Propylenglycol eignen sich als weitere mehrwertige Alkohole solche mit mindestens 2 OH-Gruppen, bevorzugt Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800, eingesetzt werden. Besonders bevorzugt ist der Einsatz von Glycerin, so dass Mittel, die außer Glycerin keine anderen mehrwertigen Alkohole enthalten, besonders bevorzugt sind.

Im Hinblick auf die Vorbehandlung vor einer oxidativen Haarbehandlung ist der Einsatz bestimmter Pflegestoffe in den Vorbehandlungsmitteln des erfindungsgemäßen Verfahrens bevorzugt.

Erfindungsgemäß bevorzugt verwendete Vorbehandlungsmittel sind dadurch gekennzeichnet, dass sie zusätzlich Pflegestoff(e) in einer Gesamtmenge von 0,001 bis 10 Gew.-%, bevorzugt 0,005 bis 7,5 Gew.-%, besonders bevorzugt 0,01 bis 5 Gew.-% und insbesondere 0,05 bis 2,5 Gew.-%, jeweils bezogen auf das Gesamtgewicht des Vorbehandlungsmittels, enthalten. Bevorzugte Pflegestoff(e) sind ausgewählt aus mindestens einer der nachfolgend genannten Gruppen:
i. L-Carnitin und/oder seiner Salze;
ii. Taurin und/oder seiner Salze;
iii. Niacinamid;
iv. Ubichinon;
v. Ectoin;
vi. Vitamine;
vii. Flavonoide.

Als weiteren Inhaltsstoff können die erfindungsgemäß verwendeten Vorbehandlungsmittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3',4'-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäß verwendete Vorbehandlungsmittel enthalten eine oder mehrere Aminosäuren in engeren Mengenbereichen. Hier sind erfindungsgemäß verwendete Vorbehandlungsmittel dadurch gekennzeichnet, dass sie als Pflegestoff 0,01 bis 5 Gew.-%, bevorzugt 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), bevorzugt aus der Gruppe Glycin und/oder Alanin und/oder Valin und/oder Lysin und/oder Leucin und/oder Threonin enthalten, jeweils bezogen auf das Gesamtgewicht des Vorbehandlungsmittels.

Das erfindungsgemäß verwendete Vorbehandlungsmittel kann als niedrig-viskose Wasser-basierte Emulsion, als Spray, als Creme, Gel, Lotion, Paste, Shampoo oder Conditioner, konfektioniert sein. Das erfindungsgemäße Verfahren umfasst das Aufbringen eines Vorbehandlungsmittels auf keratinische Fasern und eine sich innerhalb eines Zeitraumes von einer Sekunde bis 24 Stunden daran anschließende oxidative Haarbehandlung.

Ein großer Vorteil der in Schritt a) eingesetzten Vorbehandlungsmittel ist es, dass sie nicht nur dann wirksam sind, wenn sie unmittelbar vor der oxidativen Haarbehandlung angewendet werden, sondern bis zu 24 Stunden vorher angewendet werden können, ohne dass durch äußere Einflüsse eine Abschwächung der Wirkung zu befürchten wäre. Auf diese Weise ist es möglich, beispielsweise morgens nach der Haarwäsche Schritt a) des erfindungsgemäßen Verfahrens durchzuführen und die oxidative Haarbehandlung erst abends.

Erfindungsgemäß bevorzugte Verfahren sind dadurch gekennzeichnet, dass der Zeitraum zwischen den Verfahrensschritten a und b von 2 Sekunden bis 20 Minuten, bevorzugt 30 Sekunden bis 10 Minuten, besonders bevorzugt 1 bis 5 Minuten, beträgt.

Weitere erfindungsgemäß bevorzugte Verfahren sind dadurch gekennzeichnet, dass man das in Verfahrensschritt a applizierte Vorbehandlungsmittel für einen Zeitraum von 2 Sekunden bis 120 Minuten, bevorzugt 5 Sekunden bis 10 Minuten, auf das Haar einwirken lässt, bevor Verfahrensschritt b erfolgt.

Weitere erfindungsgemäß bevorzugte Verfahren sind dadurch gekennzeichnet, dass man das in Verfahrensschritt a applizierte Vorbehandlungsmittel für einen Zeitraum von 2 Sekunden bis 120 Minuten, bevorzugt 5 Sekunden bis 10 Minuten, auf das Haar einwirken lässt, bevor mindestens einer der folgenden Verfahrensschritte a)i), die vor dem Verfahrensschritt b) erfolgen, erfolgt:
Ausspülen des Haars;
Trocknen des Haars mit einem Handtuch,
Trocknen lassen des Haars an der Luft;
Trockenfönen des Haars,
Trocknen des Haars mit einer Trockenhaube,
Kombinationen der vorgenannten Verfahrensschritte.

Der Trockenvorgang erfolgt bevorzugt bei einer Temperatur von 20°C bis 150°C.

Dem Trockenvorgang bzw. den Trockenvorgängen geht besonders bevorzugt kein Ausspülen des Haars voraus. Ein erfindungsgemäß bevorzugtes Verfahren ist daher dadurch gekennzeichnet, dass zwischen der in Verfahrensschritt a erfolgten Applikation des Vorbehandlungsmittels und dem Trockenvorgang bzw. den Trockenvorgängen kein Ausspülen des Haars erfolgt. Es kann aber erfindungsgemäß auch bevorzugt sein, nach Schritt a) das Haar erst auszuspülen und anschließend zu trocknen, bevor der Behandlungsschritt b erfolgt.

Das erfindungsgemäße Verfahren ist weiterhin dadurch gekennzeichnet, dass Schritt b) das Aufbringen eines Färbe- und/oder Aufhellmittels auf die keratinischen Fasern umfasst, das durch Vermischen einer Zusammensetzung (A), die mindestens ein Alkalisierungsmittel enthält, mit einer Zusammensetzung (B), die in einem kosmetischen Träger mindestens ein Oxidationsmittel enthält, erhalten wird, wobei die Zusammensetzung (B) mindestens ein Acylpyridiniumderivat der Formel (Acylpyr-I) enthält, 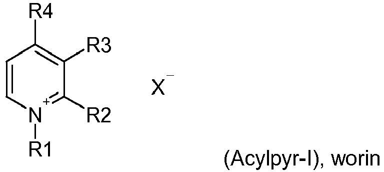
- R1: für eine C₁-C₆-Alkylgruppe, eine C₂-C₆-Alkenylgruppe, eine C₂-C₆-Hydroxy-alkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkyl-gruppe, eine Arylgruppe oder eine Heteroarylgruppe steht,
- R2, R3 und R4: jeweils unabhängig voneinander für Wasserstoff, eine C₁-C₆-Alkylgruppe, ein Halogenatom oder eine C₁-C₆-Acylgruppe steht, mit der Maßgabe, dass zumindest einer der Reste R2, R3 und R4 für eine C₁-C₆-Acylgruppe steht,
- und X⁻: für ein physiologisch verträgliches Anion steht.

Die in den erfindungsgemäßen und erfindungsgemäß bevorzugten Verfahren und Kit-of-Parts eingesetzte Zusammensetzung (B) enthält als ersten zwingenden Inhaltsstoff mindestens ein Acylpyridiniumderivat der Formel (Acylpyr-I) gemäß der vorstehenden Erläuterung. Überraschend wurde gefunden, dass ein derartiges Acylpyridiniumderivat in synergistischer Weise mit dem erfindungsgemäß verwendeten Vorbehandlungsmittel wechselwirkt und zu einer unerwarteten Reduzierung der Haarschädigung führte.

Beispiele für die genannten Substituenten der Verbindungen der Formel (Acylpyr-I) werden nachfolgend, aber nicht beschränkend genannt: Beispiele für C₁-C₆-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Beispiele für eine C₂-C₆-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe, wobei die Prop-2-enylgruppe bevorzugt ist. Beispiele für eine C₂-C₆-Hydroxyalkylgruppe sind -CH₂CH₂OH, -CH₂CH₂CH₂OH, -CH₂CH(OH)CH₃ und -CH₂CH₂CH₂CH₂OH, wobei die Gruppe -CH₂CH₂OH bevorzugt ist. Beispiele für C₁-C₆-Alkoxy-C₂-C₆-alkylgruppen sind die Gruppen -CH₂CH₂OCH₃, -CH₂CH₂CH₂OCH₃, -CH₂CH₂OCH₂CH₃, -CH₂CH₂CH₂OCH₂CH₃, -CH₂CH₂OCH(CH₃)₂, -CH₂CH₂CH₂OCH(CH₃)₂. Beispiele für eine Carboxy-C₁-C₆-alkylgruppe sind die Carboxymethylgruppe, die 2-Carboxyethylgruppe oder die 3-Carboxypropylgruppe. Beispiele für Aryl-C₁-C₆-alkylgruppen sind die Benzylgruppe und die 2-Phenylethylgruppe. Beispiele für eine Heteroaryl-C₁-C₆-alkylgruppe sind die Pyridin-2-ylmethylgruppe, die Pyridin-3-ylmethylgruppe, die Pyridin-4-ylmethylgruppe, die Pyrimidin-2-ylmethylgruppe, die Pyrrol-1-ylmethylgruppe, die Pyrrol-1-ylethylgruppe, die Pyrazol-1-ylmethylgruppe oder die Pyrazol-1-ylethylgruppe. Beispiele für eine Arylgruppe sind die Phenylgruppe, die 1-Naphthylgruppe oder die 2-Naphthylgruppe. Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-4-ylgruppe. Beispiele einer C₁-C₆-Acylgruppe sind Acetyl (1-Oxo-ethyl), 1-Oxo-propyl, 1-Oxo-butyl, 1-Oxo-Pentyl, 1-Oxo-2,2-dimethylpropyl und 1-Oxo-hexyl.

In einer Ausführungsform der vorliegenden Erfindung sind solche Verbindungen gemäß Formel (Acylpyr-I) bevorzugt, bei welchen der Rest R1 der allgemeinen Struktur (Acylpyr-I) für eine C₁-C₆-Alkylgruppe, für eine C₂-C₆-Alkenylgruppe oder für eine C₂-C₆-Hydroxyalkylgruppe steht. Es ist erfindungsgemäß bevorzugt, wenn der Rest R1 für eine C₁-C₆-Alkylgruppe, bevorzugt für Methyl, Ethyl, n-Propyl oder Isopropyl und insbesondere bevorzugt für Methyl, steht.

Es hat sich herausgestellt, dass die Acylpyridiniumderivate gemäß Formel (Acylpyr-I) erfindungsgemäß besonders vorteilhafte Eigenschaften besitzen, wenn sie die Acyl-Gruppe entweder in 2- oder 4-Position am Pyridin-Ring tragen. Bevorzugte Verbindungen der Formel (Acylpyr-I) sind weiterhin solche Verbindungen, bei denen entweder der Rest R2 oder der Rest R4 für eine C₁-C₆-Acylgruppe, bevorzugt für eine Acetylgruppe, steht. Es ist weiterhin bevorzugt, wenn einer der Reste R2 oder R4 für eine Acetylgruppe steht, während der andere dieser Reste sowie der Rest R3 jeweils für Wasserstoff stehen. Eine weitere Ausführungsform der vorliegenden Erfindung ist daher dadurch gekennzeichnet, dass das Mittel als Acylpyridiniumderivat gemäß Formel (Acylpyr-I) mindestens ein 2-Acetylpyridiniumderivat und/oder 4-Acetylpyridiniumderivat enthält.

Geeignete Acetylpyridiniumderivate sind dabei die physiologisch verträglichen Salze, die als Kation ein Acetylpyridiniumderivat, ausgewählt aus 4-Acetyl-1-methylpyridinium, 4-Acetyl-1-allylpyridinium, 4-Acetyl-1-(2-hydroxyethyl)pyridinium, 2-Acetyl-1-methylpyridinium, 2-Acetyl-1-allylpyridinium und 2-Acetyl-1-(2-hydroxyethyl)pyridinium, enthalten.

Es ist bevorzugt, wenn das Anion X⁻ gemäß Formel (Acylpyr-I) ausgewählt wird aus Halogenid, insbesondere Chlorid, Bromid und Iodid, Benzolsulfonat, p-Toluolsulfonat, C₁-C₄-Alkylsulfonat, Trifluormethansulfonat, Acetat, Triflluoracetat, Perchlorat, ½ Sulfat, Hydrogensulfat, Tetrafluorborat, Hexafluorphosphat oder Tetrachlorzinkat. Erfindungsgemäß besonders bevorzugt ist es, wenn das Anion X⁻ für Hydrogensulfat, p-Toluolsulfonat, Benzolsulfonat oder Acetat steht. Außerordentlich bevorzugt ist das Anion X⁻ p-Toluolsulfonat.

Insbesondere sind solche Verfahren und Kits erfindungsgemäß bevorzugt, die dadurch gekennzeichnet sind, dass das Acylpyridiniumderivat gemäß Formel (Acylpyr-I) ausgewählt ist aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat.

Erfindungsgemäß bevorzugte Verfahren und Kits sind dadurch gekennzeichnet, dass sie als das Acylpyridiniumderivat gemäß Formel (Acylpyr-I) eine Verbindung, ausgewählt aus 4-Acetyl-1-methylpyridinium-p-toluolsulfonat und/oder 2-Acetyl-1-methylpyridinium-p-toluolsulfonat, insbesondere 4-Acetyl-1-methylpyridinium-p-toluolsulfonat, enthalten.

Erfindungsgemäß haben sich solche Verfahren und Kits als bevorzugt herausgestellt, die dadurch gekennzeichnet sind, dass sie ein oder mehrere Acylpyridiniumderivate der Formel (Acylpyr-I) in einer Gesamtmenge von 0,001 bis 15 Gew.-%, bevorzugt von 0,01 bis 10 Gew.-% und besonders bevorzugt von 0,1 bis 5 Gew.-%, jeweils bezogen auf das Gesamtgewicht des in Verfahrensschritt b) applizierten Färbe- und/oder Aufhellmittels, enthalten.

Die in dem erfindungsgemäßen Verfahren eingesetzte Zusammensetzung (B) enthält als zweiten zwingenden Inhaltsstoff mindestens ein Oxidationsmittel. Bevorzugte Oxidationsmittel sind ausgewählt aus Peroxoverbindungen, bevorzugt ausgewählt aus Wasserstoffperoxid, festen Anlagerungsverbindung von Wasserstoffperoxid an anorganische oder organische Verbindungen, wie Natriumperborat, Natriumpercarbonat, Magnesiumpercarbonat, Natriumpercarbamid, Polyvinylpyrrolidon˙n H₂O₂ (n ist eine positive ganze Zahl größer 0), Harnstoffperoxid und Melaminperoxid, weiterhin ausgewählt aus Diammoniumperoxodisulfat (auch als Ammoniumpersulfat bezeichnet), Dinatriumperoxodisulfat (auch als Natriumpersulfat bezeichnet) und Dikaliumperoxodisulfat (auch als Kaliumpersulfat bezeichnet) sowie aus Mischungen dieser Oxidationsmittel. Erfindungsgemäß ganz besonders bevorzugt verwendete Oxidationsmittel sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; bevorzugt werden 6- bis 12-Gewichtsprozentige Lösungen in Wasser verwendet. Erfindungsgemäß bevorzugte Verfahren sind dadurch gekennzeichnet, dass die eingesetzte Zusammensetzung (B) - bezogen auf ihr Gewicht - 1 bis 24 Gew.-%, bevorzugt 4 - 10 Gew.-%, besonders bevorzugt 3 - 6 Gew.-% Wasserstoffperoxid (berechnet als 100%iges H₂O₂) enthält.

Der kosmetisch verträgliche Träger der Zusammensetzung (B) ist bevorzugt wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Zusammensetzung (B), insbesondere Ethanol bzw. Isopropanol, zu verstehen. Die erfindungsgemäß bevorzugten Zusammensetzungen (B) können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung Wasser in einer Gesamtmenge von 35 - 97 Gew.-%, besonders bevorzugt 50 - 90 Gew.-%, besonders bevorzugt 60 - 80 Gew.-%, jeweils bezogen auf das Gesamtgewicht der Zusammensetzung (B).

Erfindungsgemäß bevorzugt besitzt die Zusammensetzung (B) einen schwach sauren pH-Wert, bevorzugt einen pH-Wert von pH 2 bis pH 6, besonders bevorzugt von pH 2,5 bis pH 4,5, außerordentlich bevorzugt von pH 3,0 bis pH 4,0. Bei den pH-Werten im Sinne der vorliegenden Erfindung handelt es sich um pH-Werte, die bei einer Temperatur von 22°C gemessen wurden. Zur Einstellung des pH-Werts sind dem Fachmann gängige Acidifizierungs- und Alkalisierungsmittel geläufig. Erfindungsgemäß bevorzugte Acidifizierungsmittel sind Genuss-Säuren, wie beispielsweise Milchsäure, Zitronensäure, Essigsäure, Äpfelsäure oder Weinsäure, sowie verdünnte Mineralsäuren.

Das in Verfahrensschritt b applizierte Färbe- und/oder Aufhellmittel sollte sich durch eine sehr gute Mischbarkeit der beiden Zusammensetzungen (A) und (B) auszeichnen. Das aus der Vermischung resultierende Färbe- und/oder Aufhellmittel soll eine ausreichende Viskosität aufweisen, so dass das Mittel sich einerseits gut auftragen lässt, andererseits während der Anwendung am Wirkort verbleibt und nicht von den Fasern herabfließt.

Zur Erzielung einer ausreichenden Verdickung ist der Einsatz von polymeren Verdickungsmitteln, deren verdickende Eigenschaften sich mit dem pH-Wert ändern, bevorzugt. Diese Eigenschaft lässt sich besonders vorteilhaft nutzen, wenn der polymere Verdicker in der sauren Oxidationsmittelzusammensetzung (B) enthalten ist, da dieses Mittel beim Vermischen zum Färbe- und/oder Aufhellmittel einen großen pH-Wert-Wechsel erfährt. Daher enthält die Zusammensetzung (B) bevorzugt mindestens einen anionischen polymeren Verdicker, der bei einem alkalischen pH-Wert zu einer deutlichen Viskositätserhöhung führt. Als solche anionischen, polymeren Verdickungsmittel sind bevorzugt vernetzte, aber auch unvernetzte Homo- oder Copolymere von Acrylsäure oder Methacrylsäure besonders bevorzugt.

Weiterhin hat es sich als vorteilhaft erweisen, wenn die Zusammensetzung (B) mindestens einen Stabilisator oder Komplexbildner enthält. Besonders bevorzugte Stabilisatoren sind Phenacetin, Alkalibenzoate (Natriumbenzoat) und Salicylsäure.

Erfindungsgemäß bevorzugt ist auch der Einsatz von so genannten Komplexbildnern in der Zusammensetzung (B). Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-atomen in der Regel unter Bildung von Chelat-Komplexen reagieren, sind erfindungsgemäß einsetzbar. Die Polymergebundenen Liganden der entstehenden Metall-Komplexe können dabei aus nur einem Makromolekül stammen oder aber zu verschiedenen Polymerketten gehören. Erfindungsgemäß bevorzugte Komplexbildner sind stickstoffhaltige Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/ oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz. Ebenfalls bevorzugt ist Dipicolinsäure.

Die in dem erfindungsgemäßen Verfahren und in dem Kit-of-Parts eingesetzte Zusammensetzung (A) enthält als zwingenden Inhaltsstoff mindestens ein Alkalisierungsmittel. Oxidative Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des in Schritt b) eingesetzten Färbe- und/oder Aufhellmittels im Bereich von 7 und 11, insbesondere im Bereich von 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, basischen Aminosäuren, Alkalihydroxiden, Alkanolaminen, Alkalimetallmetasilikaten, Alkaliphosphaten und Alkalihydrogenphosphaten, ausgewählt werden. Als Alkalimetallionen dienen bevorzugt Lithium, Natrium, Kalium, insbesondere Natrium oder Kalium.

Die als Alkalisierungsmittel einsetzbaren basischen Aminosäuren werden bevorzugt ausgewählt aus der Gruppe L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als Alkalisierungsmittel einsetzbaren Alkalihydroxide werden bevorzugt ausgewählt aus der Gruppe Natriumhydroxid und Kaliumhydroxid.

Die als Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine sind ausgewählt aus der Gruppe 2-Aminoethan-1-ol, 2-Amino-2-methylpropan-1-ol und 2-Amino-2-methyl-propan-1,3-diol.

Für oxidative Färbeverfahren wird üblicherweise kurz vor der Applikation auf die Faser, insbesondere das Haar, eine Färbezusammensetzung (A), die mindestens ein Alkalisierungsmittel sowie ein oder mehrere Oxidationsfarbstoffvorprodukte und ggf. ein oder mehrere direktziehende Farbstoffe enthält, mit einer wässrigen Oxidationsmittel-haltigen Zusammensetzung (B) zu einem anwendungsbereiten Färbemittel vermischt und dann auf die Faser, insbesondere das Haar, appliziert.

Für oxidative Aufhellverfahren wird üblicherweise kurz vor der Applikation auf die Faser, insbesondere das Haar, eine Aufhellzusammensetzung (A), die mindestens ein Alkalisierungsmittel sowie ggf. ein oder mehrere direktziehende Farbstoffe und/oder ggf. ein oder mehrere Oxidationsfarbstoffvorprodukte enthält, mit einer wässrigen Oxidationsmittel-haltigen Zusammensetzung (B) zu einem anwendungsbereiten Aufhellmittel vermischt und dann auf die Faser, insbesondere das Haar, appliziert.

Meistens sind Färbe- und/oder Aufhellzusammensetzung (A) und die Oxidationsmittel-haltige Zusammensetzung (B) so aufeinander abgestimmt, dass bei einem Mischungsverhältnis von 1:1, bezogen auf Gewichtsteile, im Färbe- und/oder Aufhellmittel eine Anfangskonzentration an Wasserstoffperoxid von 0,5 bis 12 Gew.-%, bevorzugt 2 bis 10 Gew.-%, besonders bevorzugt 3 bis 6 Gew.-% Wasserstoffperoxid (berechnet als 100%iges H₂O₂), bezogen auf das Gewicht des Färbe- und/oder Aufhellmittels, vorliegt. Es ist aber genauso gut möglich, die Färbe- und/oder Aufhellzusammensetzung (A) und die Oxidationsmittel-haltige Zusammensetzung (B) so aufeinander abzustimmen, dass sich die im anwendungsbereiten Färbe- und/oder Aufhellmittel notwendigen Konzentrationen durch andere Mischungsverhältnisse als 1:1 ergeben, beispielsweise durch ein Gewichts-bezogenes Mischungsverhältnis von 1:2 oder 1:3 oder auch 2:3. Erfindungsgemäß bevorzugte Verfahren sind dadurch gekennzeichnet, dass das in Verfahrensschritt b) eingesetzte anwendungsbereite Färbe- und/oder Aufhellmittel eine Anfangsmenge an Wasserstoffperoxid von 0,5 - 12 Gew.-%, bevorzugt 2 - 10 Gew.-%, besonders bevorzugt 3 - 6 Gew.-% Wasserstoffperoxid (berechnet als 100%iges H₂O₂) bezogen auf das Gewicht des Färbe- und/oder Aufhellmittels, enthält.

Erfindungsgemäß bevorzugte Verfahren und Kits-of-Parts sind dadurch gekennzeichnet, dass das in Verfahrensschritt b) eingesetzte anwendungsbereite Färbe- und/oder Aufhellmittel mindestens ein kosmetisches Öl in einer Gesamtmenge von 5 - 50 Gew.-%, bevorzugt 8 - 40 Gew.-% besonders bevorzugt 12 - 30 Gew.-%, außerordentlich bevorzugt 15 - 25 Gew.-%, enthält, jeweils bezogen auf das Gewicht des Färbe- und/oder Aufhellmittels.

Für eine Färbung, die eine starke Aufhellung sehr dunklen Haares erfordert, ist der Einsatz von Wasserstoffperoxid oder dessen Anlagerungsprodukten an organische bzw. anorganische Verbindungen oftmals nicht ausreichend. In diesen Fällen wird in der Regel eine Kombination aus Wasserstoffperoxid und Peroxodisulfatsalzen (Persulfatsalzen) eingesetzt. Bevorzugte Persulfatsalze sind Ammoniumperoxidisulfat, Kaliumperoxidisulfat, Natriumperoxidisulfat, sowie Mischungen hiervon.

Das mindestens eine Persulfatsalz ist bevorzugt in einer Gesamtmenge von 0,1 bis 25 Gew.-%, besonders bevorzugt in einer Gesamtmenge von 1 bis 15 Gew.-%, bezogen auf das Gewicht des anwendungsbereiten Färbemittels, enthalten.

Weitere erfindungsgemäß bevorzugte Verfahren und Kits-of-Parts sind dadurch gekennzeichnet, dass die Zusammensetzung (B) mindestens ein kosmetisches Öl in einer Gesamtmenge von 10 - 80 Gew.-%, bezogen auf das Gewicht der Zusammensetzung (B). Das kosmetische Öl ist unter Normalbedingungen (20 °C, 1013,25 mbar) flüssig; ätherische Öle und Parfümöle bzw. Riechstoffe werden nicht zu den kosmetischen Ölen gezählt. Die unter Normalbedingungen flüssigen kosmetischen Öle sind mit Wasser nicht mischbar. Unter ätherischen Ölen werden erfindungsgemäß Gemische aus flüchtigen Komponenten verstanden, die durch Wasserdampfdestillation aus pflanzlichen Rohstoffen hergestellt werden, wie z. B. Citrusöle. Sofern in der vorliegenden Anmeldung von einem kosmetischen Öl die Rede ist, handelt es sich hierbei immer um ein kosmetisches Öl, das kein Riechstoff und kein ätherisches Öl ist, unter Normalbedingungen flüssig und mit Wasser nicht mischbar ist.

Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/ oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Erfindungsgemäß bevorzugte kosmetische Öle sind ausgewählt aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan (erhältlich z. B. unter dem Handelsnamen Cetiol^{®}S von BASF).

Weitere erfindungsgemäß bevorzugte kosmetische Öle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.

Weitere erfindungsgemäß bevorzugte kosmetische Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol^{®} G 16), 2-Octyldodecanol (Eutanol^{®} G), 2-Ethylhexylalkohol und Isostearylalkohol.

Weitere bevorzugte kosmetische Öle sind ausgewählt aus Mischungen aus Guerbetalkoholen und Guerbetalkoholestern, z.B. dem Handelsprodukt Cetiol^{®} PGL (2-Hexyldecanol und 2-Hexyldecyllaurat).

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C₈₋₃₀-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol^{®} 318, Myritol^{®} 331 (BASF) oder Miglyol^{®} 812 (Hüls) mit unverzweigten Fettsäureresten sowie Glyceryltrüsostearin mit verzweigten Fettsäureresten.

Weitere erfindungsgemäß besonders bevorzugte kosmetische Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte kosmetische Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen 2-Hexyldecylstearat (Eutanol^{®} G 16 S), 2-Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte kosmetische Öle sind ausgewählt aus den Anlagerungsprodukten von 1 bis 5 Propylenoxid-Einheiten an ein- oder mehrwertige C₈₋₂₂-Alkanole, wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat.

Weitere erfindungsgemäß bevorzugte kosmetische Öle sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat (Cetiol^{®} CC) oder die Ester gemäß der Lehre der DE 19756454 A1, insbesondere Glycerincarbonat.

Weitere kosmetische Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt.

Es kann erfindungsgemäß außerordentlich bevorzugt sein, Mischungen der vorgenannten kosmetischen Öle einzusetzen.

Bevorzugte erfindungsgemäß verwendete Zusammensetzungen (B) sind dadurch gekennzeichnet, dass das kosmetische Öl ausgewählt ist aus natürlichen und synthetischen Kohlenwasserstoffen, besonders bevorzugt aus Paraffinölen, C₁₈-C₃₀-Isoparaffinen, insbesondere Isoeicosan, Polyisobutene und Polydecene, C₈-C₁₆-Isoparaffinen, sowie 1,3-Di-(2-ethylhexyl)-cyclohexan; den Benzoesäureestern von linearen oder verzweigten C₈₋₂₂-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten C₈₋₃₀-Fettsäuren, insbesondere natürlichen Ölen; den Dicarbonsäureestern von linearen oder verzweigten C₂-C₁₀-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige C₈₋₂₂-Alkanole; den Anlagerungsprodukten von mindestens 6 Ethylenoxid- und/oder Propylenoxid-Einheiten an ein- oder mehrwertige C₃₋₂₂-Alkanole; den C₈-C₂₂-Fettalkoholestern einwertiger oder mehrwertiger C₂-C₇-Hydroxycarbonsäuren; den symmetrischen, unsymmetrischen oder cyclischen Estern der Kohlensäure mit C₃₋₂₂-Alkanolen, C₃₋₂₂-Alkandiolen oder C₃₋₂₂-Alkantriolen; den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen; Siliconölen sowie Mischungen der vorgenannten Substanzen.

Bevorzugte erfindungsgemäße Verfahren und Kits-of-Parts sind dadurch gekennzeichnet, dass die erfindungsgemäß verwendete Zusammensetzung (B) mindestens ein kosmetisches Öl in einer Gesamtmenge von 12 - 70 Gew.-%, bevorzugt 14 - 60 Gew.-%, besonders bevorzugt 15 - 52 Gew.-% und außerordentlich bevorzugt 17 - 35 Gew.-%, enthält, jeweils bezogen auf das Gewicht der Zusammensetzung (B).

Weitere bevorzugte erfindungsgemäße Verfahren und Kits-of-Parts sind dadurch gekennzeichnet, dass die erfindungsgemäß verwendete Zusammensetzung (B) mindestens ein Tensid enthält.

Bei der Auswahl erfindungsgemäß geeigneter Tenside ist es besonders bevorzugt, ein Gemisch von Tensiden einzusetzen, um die Stabilität der erfindungsgemäß verwendeten Oxidationsmittel-Zusammensetzungen (B) optimal einstellen zu können.

Bevorzugte erfindungsgemäße Verfahren und Kits-of-Parts sind dadurch gekennzeichnet, dass das in der Zusammensetzung (B) enthaltene Tensid ausgewählt ist aus nichtionischen Tensiden und anionischen Tensiden sowie aus Mischungen hiervon. Besonders bevorzugt verwendete nichtionische Tenside sind ausgewählt sind aus mit 20 - 100 Mol Ethylenoxid pro Mol ethoxyliertem Rizinusöl, ethoxylierten C₈-C₂₄-Alkanolen mit 10- 100 Mol Ethylenoxid pro Mol, ethoxylierten C₈-C₂₄-Carbonsäuren mit 10 - 100 Mol Ethylenoxid pro Mol, mit 20 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Alkylmono- und -oligoglycosiden mit 8 bis 22 Kohlenstoffatomen im Alkylrest und deren ethoxylierten Analoga, sowie Mischungen der vorgenannten Substanzen.

Bevorzugt ist mit 40 - 80 Mol Ethylenoxid pro Mol ethoxyliertes Rizinusöl in den erfindungsgemäß bevorzugt verwendeten Zusammensetzungen (B) enthalten.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, bevorzugt 10 - 30, besonders bevorzugt 15 bis 25 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet. Besonders bevorzugt sind Laureth-10, Laureth-12, Laureth-15, Laureth-20, Laureth-30, Myreth-10, Myreth-12, Myreth-15, Myreth-20, Myreth-30, Ceteth-10, Ceteth-12, Ceteth-15, Ceteth-20, Ceteth-30, Steareth-10, Steareth-12 Steareth-15, Steareth-20, Steareth-30, Oleth-10, Oleth-12, Oleth-15, Oleth-20, Oleth-30, Ceteareth-10, Ceteareth-15, Ceteareth-12, Ceteareth-15, Ceteareth-20, Ceteareth-30 sowie Coceth-10, Coceth-12, Coceth-15, Coceth-20 und Coceth-30.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, bevorzugt 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12-18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Bevorzugte mit 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80.

Weiterhin werden bevorzugt C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, bevorzugt 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 -2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Als anionische Tenside eignen sich in den erfindungsgemäß verwendeten Zusammensetzungen (B) alle für die Verwendung am menschlichen Körper geeigneten anionischen oberflächenaktiven Stoffe, die eine wasserlöslich machende, anionische Gruppe, beispielsweise eine Carboxylat-, Sulfat-, Sulfonat- oder Phosphat-Gruppe, und eine lipophile Alkylgruppe mit etwa 8 bis 30 C-Atomen, bevorzugt 8 bis 24 C-Atomen im Molekül aufweisen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen), polyethoxylierte Ethercarbonsäuren, Acylsarcoside, Acyltauride, Acylisethionate, Sulfobernsteinsäuremono- und - dialkylester und Sulfobernsteinsäuremono-alkylpolyoxyethylester mit 1 bis 6 Ethylenoxidgruppen, lineare Alkansulfonate, lineare alpha-Olefinsulfonate, Sulfonate ungesättigter Fettsäuren mit bis zu 6 Doppelbindungen, alpha-Sulfofettsäuremethylester von Fettsäuren, C₈-C₂₀-Alkylsulfate und C₈-C₂₀-Alkylethersulfate mit zu bis 15 Oxyethylgruppen, Gemische oberflächenaktiver Hydroxysulfonate, sulfatierte Hydroxyalkylpolyethylen- und/oder Hydroxyalkylenpropylenglykolether, Ester von Weinsäure oder Zitronensäure mit ethoxylierten oder propoxylierten Fettalkoholen, gegebenenfalls polyethoxylierte Alkyl- und/oder Alkenyletherphosphate, sulfatierte Fettsäurealkylenglykolester, sowie Monoglyceridsulfate und Monoglyceridethersulfate.

Bevorzugte anionische Tenside sind Seifen, C₈-C₂₀-Alkylsulfate, C₈-C₂₀-Alkylethersulfate und C₈-C₂₀-Ethercarbonsäuren mit 8 bis 20 C-Atomen in der Alkylgruppe und bis zu 12 Ethylenoxidgruppen im Molekül. Besonders bevorzugt ist Natriumcetearylsulfat.

Bevorzugt beträgt die Gesamtmenge an mindestens einem Tensid in der Oxidationsmittelzusammensetzung (B) 0,1 - 5 Gew.-%, bevorzugt 0,5 - 3 Gew.-% und besonders bevorzugt 1 - 2 Gew.-%, jeweils bezogen auf das Gesamtgewicht der Oxidationsmittelzusammensetzung (B).

Besonders bevorzugt enthält die erfindungsgemäß verwendete Oxidationsmittelzusammensetzung (B) insgesamt 0,1 bis 5 Gew.-%, bevorzugt 0,5 bis 3 Gew.-% und besonders bevorzugt 1 - 2 Gew.-%, jeweils bezogen auf das Gesamtgewicht der Oxidationsmittelzusammensetzung (B), einer Mischung aus nichtionischen und anionischen Tensiden.

Weitere bevorzugte erfindungsgemäße Verfahren sind dadurch gekennzeichnet, dass die erfindungsgemäß verwendete Zusammensetzung (B) mindestens ein lineares gesättigtes Alkanol mit 12 - 30 Kohlenstoffatomen enthält.

Bevorzugte lineare gesättigte Alkanole mit 12 - 30 Kohlenstoffatomen, insbesondere mit 16 - 22 Kohlenstoffatomen, sind ausgewählt aus Cetylalkohol, Stearylalkohol, Arachidylalkohol, Behenylalkohol und Lanolinalkohol sowie Mischungen dieser Alkanole. Erfindungsgemäß besonders bevorzugte Alkanolmischungen sind solche, wie sie bei der technischen Hydrierung von pflanzlichen und tierischen Fettsäuren erhältlich sind. Bevorzugt beträgt die Gesamtmenge an mindestens einem linearen gesättigten Alkanol mit 12 - 30 Kohlenstoffatomen in der Oxidationsmittelzusammensetzung (B) 0,1 - 10 Gew.-%, bevorzugt 0,5 - 7 Gew.-% und besonders bevorzugt 3 - 5 Gew.-%, jeweils bezogen auf das Gesamtgewicht der Oxidationsmittelzusammensetzung (B).

Weitere bevorzugte erfindungsgemäße Verfahren und Kits-of-Parts sind dadurch gekennzeichnet, dass die Zusammensetzung (B):
1 bis 24 Gew.-%, bevorzugt 4 - 10 Gew.-%, besonders bevorzugt 3 - 6 Gew.-% Wasserstoffperoxid (berechnet als 100%iges H₂O₂), weiterhin
   mindestens ein kosmetisches Öl in einer Gesamtmenge von 12 - 70 Gew.-%, bevorzugt 14 - 60 Gew.-%, besonders bevorzugt 15 - 52 Gew.-% und außerordentlich bevorzugt 17 - 35 Gew.-%, weiterhin mindestens ein Tensid in einer Gesamtmenge von 0,1 bis 5 Gew.-%, bevorzugt 0,5 bis 3 Gew.-% und besonders bevorzugt 1 bis 2 Gew.-%, sowie
   mindestens ein lineares gesättigtes Alkanol mit 12 - 30 Kohlenstoffatomen in einer Gesamtmenge von 0,1 bis 10 Gew.-%, bevorzugt 0,5 bis 7 Gew.-% und besonders bevorzugt 3 bis 5 Gew.-%, enthält, wobei sich alle Gew.-%-Angaben auf das Gewicht der Zusammensetzung (B) beziehen.

Als für Färbeverfahren zwingende, für Aufhellverfahren optionale, Inhaltsstoffe enthält die in dem erfindungsgemäßen Verfahren eingesetzte Zusammensetzung (A) mindestens ein Oxidationsfarbstoffvorprodukt vom Entwicklertyp und mindestens ein Oxidationsfarbstoffvorprodukt vom Kupplertyp.

Oxidationsfarbstoffvorprodukte können aufgrund ihres Reaktionsverhaltens in zwei Kategorien eingeteilt werden, so genannte Entwicklerkomponenten und Kupplerkomponenten.

Kupplerkomponenten bilden im Rahmen der oxidativen Färbung allein keine signifikante Färbung aus, sondern benötigen stets die Gegenwart von Entwicklerkomponenten. Entwicklerkomponenten können mit sich selbst den eigentlichen Farbstoff ausbilden.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride oder Hydrobromide oder der Sulfate einzusetzen.

Überraschend wurde festgestellt, dass mit dem erfindungsgemäßen Verfahren unter Verwendung von mindestens einem Oxidationsfarbstoffvorprodukt vom Entwicklertyp und mindestens einem Oxidationsfarbstoffvorprodukt vom Kupplertyp Haarfärbungen mit besonders hoher Waschechtheit erzielt werden konnten.

Besonders bevorzugte Entwicklerkomponenten sind ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, den physiologisch verträglichen Salzen dieser Verbindungen sowie Mischungen dieser Entwicklerkomponenten und Entwicklerkomponentensalze.

Ganz besonders bevorzugte Entwicklerkomponenten sind ausgewählt aus 4,5-Diamino-1-(2-hydroxyethyl)pyrazol, p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin und Mischungen dieser Verbindungen sowie deren physiologisch verträglichen Salzen. Außerordentlich bevorzugt ist 4,5-Diamino-1-(2-hydroxyethyl)pyrazol und dessen physiologisch verträglichen Salze.

Die Entwicklerkomponenten werden bevorzugt in einer Gesamtmenge von 0,01 - 20 Gew.-%, besonders bevorzugt 0,2 - 10 Gew.-%, und außerordentlich bevorzugt 0,6 - 5 Gew.-%, jeweils bezogen auf das Gewicht der Zusammensetzung (A), verwendet.

Die Entwicklerkomponenten werden bevorzugt in einer Gesamtmenge von 0,005 - 10 Gew.-%, besonders bevorzugt 0,1 - 5 Gew.-%, und außerordentlich bevorzugt 0,3 - 2,5 Gew.-%, jeweils bezogen auf das Gewicht des anwendungsbereiten Färbemittels, verwendet.

Unter dem Begriff "anwendungsbereites Färbe- und oder Aufhellmittel" wird im Sinne dieser Anmeldung die Mischung aus der Zusammensetzung (A) und der Zusammensetzung (B) verstanden. Ein geeigneter kosmetischer Träger für die Zusammensetzung (A) ist insbesondere eine Cremebasis. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt cyclische Verbindungen, die am Cyclus mindestens zwei Gruppen tragen, ausgewählt aus (i) gegebenenfalls substituierten Aminogruppen und/oder (ii) Hydroxylgruppen. Wenn die cyclische Verbindung ein Sechsring (bevorzugt aromatisch) ist, so befinden sich die besagten Gruppen bevorzugt in ortho-Position oder meta-Position zueinander.

Bevorzugte erfindungsgemäße Verfahren sind dadurch gekennzeichnet, dass das mindestens eine Oxidationsfarbstoffvorprodukt vom Kupplertyp ausgewählt ist aus einer der folgenden Klassen:
- 3-Aminophenol (m-Aminophenol) und/oder dessen Derivate,
- 3-Aminoanilin (m-Diaminobenzol) und/oder dessen Derivate,
- 2-Aminoanilin (1,2-Diaminobenzol; o-Diaminobenzol) und/oder dessen Derivate,
- 2-Aminophenol (o-Aminophenol) und/oder dessen Derivate,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin,
Gemische aus zwei oder mehreren Verbindungen aus einer oder mehreren dieser Klassen sind im Rahmen dieser Ausführungsform ebenso erfindungsgemäß bevorzugt.

Erfindungsgemäß besonders bevorzugte zusätzliche Kupplerkomponenten sind ausgewählt aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol (= 2-Amino-4-Hydroxyethylaminoanisol), 1,3-Bis (2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Ganz besonders bevorzugt sind dabei 3-Aminophenol, Resorcin, 2-Methylresorcin, 5-Amino-2-methylphenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie deren physiologisch verträglichen Salze und Mischungen der genannten Komponenten.

Die mindestens eine Kupplerkomponente wird bevorzugt in einer Gesamtmenge von 0,01 - 20 Gew.-%, besonders bevorzugt 0,2 - 10 Gew.-%, und außerordentlich bevorzugt 0,6 - 5 Gew.-%, jeweils bezogen auf das Gewicht der Zusammensetzung (A), verwendet.

Die mindestens eine Kupplerkomponente wird bevorzugt in einer Gesamtmenge von 0,005 - 10 Gew.-%, bevorzugt 0,1 - 5 Gew.-%, und außerordentlich bevorzugt 0,3 - 2,5 Gew.-%, jeweils bezogen auf das Gewicht des anwendungsbereiten Oxidationsfärbemittels, verwendet.

Im Rahmen der vorliegenden Erfindung sind folgende Kombinationen aus Oxidationsfarbstoffvorprodukten vom Entwicklertyp und vom Kupplertyp besonders bevorzugt, wobei die Aminverbindungen und die Stickstoffheterocyclen auch in Form ihrer physiologisch verträglichen Salze vorliegen können:
p-Toluylendiamin / Resorcin;
p-Toluylendiamin / 2-Methylresorcin;
p-Toluylendiamin / 5-Amino-2-methylphenol;
p-Toluylendiamin / 3-Aminophenol;
p-Toluylendiamin / 2-(2,4-Diaminophenoxy)ethanol;
p-Toluylendiamin / 1,3-Bis(2,4-diaminophenoxy)propan;
p-Toluylendiamin / 1-Methoxy-2-amino-4-(2-hydroxyethylamino)benzol;
p-Toluylendiamin / 2-Amino-3-hydroxypyridin;
p-Toluylendiamin / 1-Naphthol;
2-(2-Hydroxyethyl)-p-phenylendiamin / Resorcin;
2-(2-Hydroxyethyl)-p-phenylendiamin / 2-Methylresorcin;
2-(2-Hydroxyethyl)-p-phenylendiamin / 5-Amino-2-methylphenol;
2-(2-Hydroxyethyl)-p-phenylendiamin / 3-Aminophenol;
2-(2-Hydroxyethyl)-p-phenylendiamin / 2-(2,4-Diaminophenoxy)ethanol;
2-(2-Hydroxyethyl)-p-phenylendiamin / 1,3-Bis(2,4-diaminophenoxy)propan;
2-(2-Hydroxyethyl)-p-phenylendiamin / 1-Methoxy-2-amino-4-(2-hydroxyethylamino)benzol;
2-(2-Hydroxyethyl)-p-phenylendiamin / 2-Amino-3-hydroxypyridin;
2-(2-Hydroxyethyl)-p-phenylendiamin / 1-Naphthol;
2-Methoxymethyl-p-phenylendiamin / Resorcin;
2-Methoxymethyl-p-phenylendiamin / 2-Methylresorcin;
2-Methoxymethyl-p-phenylendiamin / 5-Amino-2-methylphenol;
2-Methoxymethyl-p-phenylendiamin / 3-Aminophenol;
2-Methoxymethyl-p-phenylendiamin / 2-(2,4-Diaminophenoxy)ethanol;
2-Methoxymethyl-p-phenylendiamin / 1,3-Bis(2,4-diaminophenoxy)propan;
2-Methoxymethyl-p-phenylendiamin / 1-Methoxy-2-amino-4-(2-hydroxyethylamino)benzol;
2-Methoxymethyl-p-phenylendiamin / 2-Amino-3-hydroxypyridin;
2-Methoxymethyl-p-phenylendiamin / 1-Naphthol;
N-(4-Amino-3-methylphenyl)-N-[3-(1H-imidazol-1-yl)propyl]amin / Resorcin;
N-(4-Amino-3-methylphenyl)-N-[3-(1H-imidazol-1-yl)propyl]amin / 2-Methylresorcin;
N-(4-Amino-3-methylphenyl)-N-[3-(1H-imidazol-1-yl)propyl]amin / 5-Amino-2-methylphenol;
N-(4-Amino-3-methylphenyl)-N-[3-(1H-imidazol-1-yl)propyl]amin / 3-Aminophenol;
N-(4-Amino-3-methylphenyl)-N-[3-(1H-imidazol-1-yl)propyl]amin / 2-(2,4-Diaminophenoxy)ethanol;
N-(4-Amino-3-methylphenyl)-N-[3-(1H-imidazol-1-yl)propyl]amin / 1,3-Bis(2,4-diaminophenoxy)-propan;
N-(4-Amino-3-methylphenyl)-N-[3-(1H-imidazol-1-yl)propyl]amin / 1-Methoxy-2-amino-4-(2-hydroxyethylamino)benzol;
N-(4-Amino-3-methylphenyl)-N-[3-(1H-imidazol-1-yl)propyl]amin / 2-Amino-3-hydroxypyridin;
N-(4-Amino-3-methylphenyl)-N-[3-(1H-imidazol-1-yl)propyl]amin 1-Naphthol;
4,5-Diamino-1-(2-hydroxyethyl)pyrazol / Resorcin;
4,5-Diamino-1-(2-hydroxyethyl)pyrazol / 2-Methylresorcin;
4,5-Diamino-1-(2-hydroxyethyl)pyrazol / 5-Amino-2-methylphenol;
4,5-Diamino-1-(2-hydroxyethyl)pyrazol / 3-Aminophenol;
4,5-Diamino-1-(2-hydroxyethyl)pyrazol / 2-(2,4-Diaminophenoxy)ethanol;
4,5-Diamino-1-(2-hydroxyethyl)pyrazol / 1,3-Bis(2,4-diaminophenoxy)propan;
4,5-Diamino-1-(2-hydroxyethyl)pyrazol / 1-Methoxy-2-amino-4-(2-hydroxyethylamino)benzol;
4,5-Diamino-1-(2-hydroxyethyl)pyrazol / 2-Amino-3-hydroxypyridin;
4,5-Diamino-1-(2-hydroxyethyl)pyrazol / 1-Naphthol.

Erfindungsgemäß besonders bevorzugt sind die Kombinationen 4,5-Diamino-1-(2-hydroxyethyl)-pyrazol/3-Aminophenol und p-Toluylendiamin/3-Aminophenol. Außerordentlich bevorzugt, besonders im Hinblick auf die Verbesserung der Waschechtheit, ist die Kombination 4,5-Diamino-1-(2-hydroxyethyl)pyrazol/3-Aminophenol.

Um eine ausgewogene und subtile Nuancenausbildung zu erzielen, ist es erfindungsgemäß bevorzugt, wenn weitere farbgebende Komponenten in dem Färbemittel enthalten sind, das in dem erfindungsgemäßen Verfahren eingesetzt wird.

In einer weiteren Ausführungsform können die in Schritt b) dieser Variante des erfindungsgemäßen Verfahrens eingesetzten Mittel zusätzlich mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Ein weiteres bevorzugtes erfindungsgemäßes Verfahren ist dadurch gekennzeichnet, dass das in Schritt b applizierte Färbe- und oder Aufhellmittel nach einer Zeit von 5-60 Minuten, bevorzugt 15 - 50 Minuten, besonders bevorzugt 30 - 45 Minuten, von den Fasern abgespült wird.

Das im erfindungsgemäßen Verfahren in Schritt b eingesetzte Färbemittel wird aus einem Zweikomponentenmittel hergestellt, wobei eine Komponente, nämlich die Zusammensetzung (A), mindestens ein Alkalisierungsmittel, mindestens ein Acylpyridinium-Derivat der Formel (Acylpyr-I) und ggf. die Oxidationsfarbstoffvorprodukte und/oder Direktzieher enthält, und die andere Komponente, nämlich die Zusammensetzung (B), das oder die Oxidationsmittel enthält. Das anwendungsbereite Färbe- und/oder Aufhellmittel für Schritt b wird dann durch Mischen beider Komponenten direkt vor dem Applikationsschritt b hergestellt. Eine Auftrennung in Mehrkomponentensysteme bietet sich insbesondere dort an, wo Inkompatibilitäten der Inhaltsstoffe zu erwarten oder zu befürchten sind.

Ein weiterer Gegenstand der vorliegenden Erfindung ist daher eine Mehrkomponentenverpackungseinheit (Kit-of-Parts), umfassend
(i) einen ersten Container (C1) mit einem Mittel, enthaltend mindestens ein 4-Morpholinomethyl-substituiertes Silikon der Formel (V), 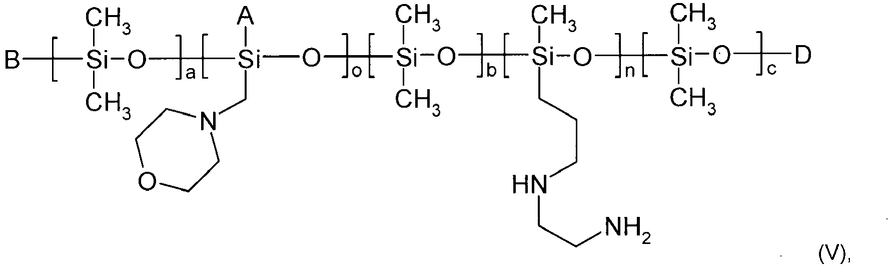 in der
   - A: für eine über ein -O- gebundene Struktureinheit (I), (II), (IIIa), (IIIb), (IIIe) oder (IIIf) 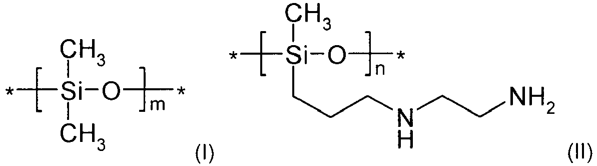 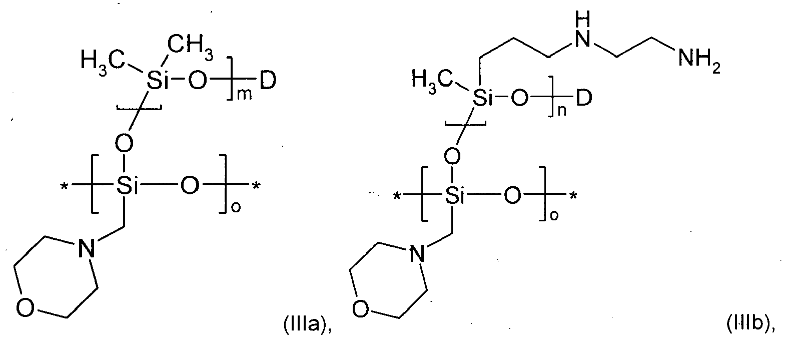 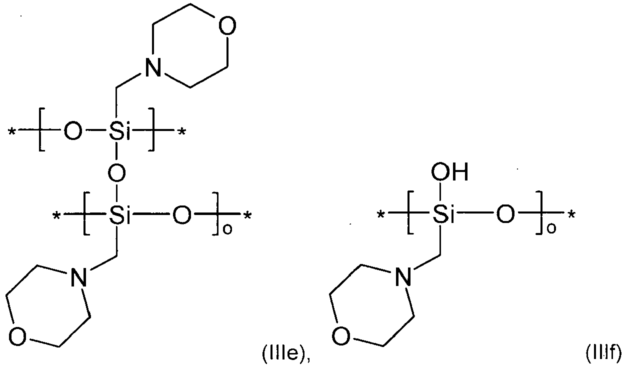 oder für einen über ein -O- gebundenen oligomeren oder polymeren Rest, enthal-tend Struktureinheiten der Formeln (I), (II), (IIIa), (IIIb), (IIIe) oder (IIIf) oder für -OH steht,
   - *: für eine Bindung zu einer der Struktureinheiten (I), (II), (IIIa), (IIIb), (IIIe) oder (IIIf) oder für eine Endgruppe B (Si-gebunden) oder D (O-gebunden) steht,
   - D: für eine Gruppe -H, -Si(CH₃)₃, -Si(CH₃)₂OH, -Si(CH₃)₂OCH₃ steht,
   - a, b und c: für ganze Zahlen von 0 bis 990 stehen, mit der Maßgabe a + b + c > 0
   - m, n und o: für ganze Zahlen von 1 bis 990 stehen,
   weiterhin
(ii) einen zweiten Container (C2) mit einer Zusammensetzung (A), enthaltend mindestens ein Alkalisierungsmittel
(iii) und weiterhin einen dritten Container (C3) mit einer Zusammensetzung (B), enthaltend in einem kosmetischen Träger mindestens ein Oxidationsmittel und mindestens ein Acylpyridiniumderivat der Formel (Acylpyr-I), 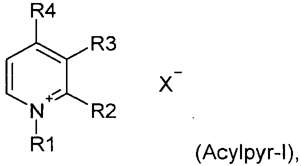 worin
   - R1: für eine C₁-C₆-Alkylgruppe, eine C₂-C₆-Alkenylgruppe, eine C₂-C₆-Hydroxy-alkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
   - R2, R3 und R4: jeweils unabhängig voneinander für Wasserstoff, eine C₁-C₆-Alkylgruppe, ein Halogenatom oder eine C₁-C₆-Acylgruppe steht, mit der Maßgabe, dass zumindest einer der Reste R2, R3 und R4 für eine C₁-C₆-Acylgruppe steht,
   - und X⁻: für ein physiologisch verträgliches Anion steht.

Das vorstehend Gesagte zu bevorzugten Ausführungsformen des erfindungsgemäßen Verfahrens gilt mutatis mutandis auch für die erfindungsgemäßen Mehrkomponentenverpackungseinheiten.

### Ausführungsbeispiele

### Beispiel 1

### Vorbehandlung mit 4-Morpholinomethyl-substituierten Silikonen der Formel (V)

Haarsträhnen wurden eine Minute lang in eine wässrige Emulsion eines 4-Morpholinomethyl-substituierten Silikons der Formel (V), die 0,01 Gew.-% 4-Morpholinomethyl-substituierte(s) Silikon(e) der Formel (V) und 0,005 Gew.-% verzweigtes Trideceth-5, weiterhin 0,006 Gew.-% Glycerin und Wasser ad 100 Gew.-% enthielt, eingetaucht und anschließend trocken gefönt.

### Herstellung der in Verfahrensschritt b applizierten Färbemittel

**Tabelle 1: Zusammensetzung (A): Färbecreme (Mengenangaben in Gew.-%):**

| | |
|---|---|
| Toluene-2,5-Diamine Sulfate | 0,02 |
| 2-Amino-4-Hydroxyethylaminoanisole Sulfate (1-Methoxy-2-amino-4-(2'-hydroxyethylamino)benzolsulfat) | 0,02 |
| 4-Amino-2-Hydroxytoluene | 0,01 |
| Cetearyl Alcohol | 14 |
| Glyceryl Stearate | 1,4 |
| Ammonium Hydroxide | 6,8 |
| Ceteareth-20 | 3,5 |
| Octyldodecanol | 1 |
| Sodium Laureth Sulfate | 0,5 |
| 1,3-Butylenglycol | 3,5 |
| Sodium Cetearyl Sulfate | 1,0 |
| Oleic Acid | 0,1 |
| Parfum (Fragrance) | 0,5 |
| Potassium Stearate | 0,5 |
| Sodium Sulfite | 0,2 |
| Tetrasodium EDTA | 0,3 |
| Carbomer | 0,3 |
| Polyquaternium-39 (ex Merquat 3330) | 0,05 |
| Potassium Hydroxide | 0,08 |
| Ascorbic Acid | 0,02 |
| Linoleamidopropyl PG-Dimonium Chloride Phosphate | 0,1 |
| Sodium Sulfate | 0,1 |
| Citric Acid | 0,002 |
| Cl 77891 (Titanium Dioxide) | 0,3 |
| Aqua (Water, Eau) | ad 100 |

Die vorstehend aufgeführte Zusammensetzung (A) wurde im Gewichtsverhältnis 1:1 entweder mit der in Tabelle 2 dargestellten Zusammensetzung (B)-1 = Vergleichs-Oxidationsmittelzusammensetzung (B) oder mit der in Tabelle 2 dargestellten Zusammensetzung (B)-2 = Oxidationsmittelzusammensetzung (B) mit erfindungsgemäßem Gehalt an Acylpyridiniumderivat jeweils zu einem anwendungsfertigen Färbemittel vermischt. Anschließend wurde das anwendungsbereite Farbemittel auf die Teststrähnen appliziert, und zwar je 4 g Färbemittel pro Gramm Haar. Dabei wurden zum einen Teststrähnen verwendet, die zuvor mit dem 4-Morpholinomethyl-substituierten Silikon der Formel (V) behandelt worden waren, zum anderen wurden unbehandelte Teststrähnen eingesetzt.

Das Färbemittel verblieb jeweils 30 Minuten auf den Strähnen. Anschließend wurden die Strähnen mit 32°C warmem Leitungswasser mit einer Fließgeschwindigkeit von 0,5 Liter pro Minute 2 Minuten lang ausgespült. Anschließend wurden die Strähnen dreimal gekämmt, bevor die Kämmbarkeits-Messungen (10 Kammstriche an jeweils 20 Strähnen) durchgeführt wurden.

**Tabelle 2: Oxidationsmittel-Zusammensetzungen (B) (Mengenangaben in Gew.-%):**

| | (B)-1 | (B)-2 |
|---|---|---|
| 4-Acetyl-1-methylpyridinium-p-toluolsulfonat | -- | 4,0 |
| Paraffinum Liquidum | 0,5 | 0,5 |
| Cetearylalkohol | 4,0 | 4,0 |
| Dipicolinsäure | 0,1 | 0,1 |
| Disodium Pyrophosphate | 0,1 | 0,1 |
| Kaliumhydroxid | 0,1 | 0,1 |
| 1,2-Propylenglycol | 1,0 | 1,0 |
| 1-Hydroxyethane-1,1-diphosphonic acid (Etidronic acid) | 0,1 | 0,1 |
| STEARTRIMONIUM CHLORIDE | 0,5 | 0,5 |
| Ceteareth-20 | 1,0 | 1,0 |
| H₂O₂ (Aktivgehalt) | 12,0 | 12,0 |
| Wasser | ad 100 | ad 100 |

**Tabelle 3: Nasskämmbarkeit; Kämmarbeit in mJ**

| | Kämmarbeit [mJ] | Relative Änderung [%] |
|---|---|---|
| (A) + (B)-1, ohne Verfahrensschritt a (Vergleich) | 1282 | 100 |
| (A) + (B)-2, ohne Verfahrensschritt a (Vergleich) | 1257 | 98 |
| (A) + (B)-1, mit Verfahrensschritt a (Vergleich) | 802 | 63 |
| (A) + (B)-2, mit Verfahrensschritt a (erfindungsgemäß) | 705 | 55 |

**Tabelle 4: Splissanzahl nach 20000 Kämmungen (Anteil in %)**

| | Splissanzahl [%] | Relative Änderung [%] |
|---|---|---|
| (A) + (B)-1, ohne Verfahrensschritt a (Vergleich) | 1,5 | 100 |
| (A) + (B)-2, ohne Verfahrensschritt a (Vergleich) | 1,5 | 100 |
| (A) + (B)-1, mit Verfahrensschritt a (Vergleich) | 1,3 | 87 |
| (A) + (B)-2, mit Verfahrensschritt a (erfindungsgemäß) | 1,1 | 73 |

Wie die in den Tabellen 3 und 4 dargestellten Daten zeigen, wirken sich sowohl die Vorbehandlung mit einem 4-Morpholinomethyl-substituierten Silikon der Formel (V) allein als auch die Verwendung einer Oxidationsmittelzusammensetzung (B) mit einem Gehalt an Acylpyridiniumderivat allein bereits positiv auf den Schutz des Haares vor oxidativer Schädigung aus: sowohl die Kämmarbeit wird um 20 bzw. 27 % reduziert, wobei eine geringere Kämmarbeit gleichbedeutend ist mit einer geringeren Haarschädigung; als auch die durch ein standardisiertes Testkämmen verursachte Splisszahl wird um 28 bzw. 39 % reduziert.

Demgegenüber bringt das erfindungsgemäße Verfahren, das die Vorbehandlung mit dem 4-Morpholinomethyl-substituierten Silikon der Formel (V) mit der Verwendung einer Oxidationsmittelzusammensetzung (B) mit einem Gehalt an Acylpyridiniumderivat kombiniert, eine weitere sehr deutliche Reduktion der Nasskämmarbeit und der Splissrate.

## Patentansprüche

1. Verfahren zur oxidativen Aufhellung und/oder Färbung keratinischer Fasern, insbesondere von Haaren, **dadurch gekennzeichnet, dass**
a) ein Vorbehandlungsmittel, das mindestens ein 4-Morpholinomethyl-substituiertes Silikon der Formel (V) enthält, 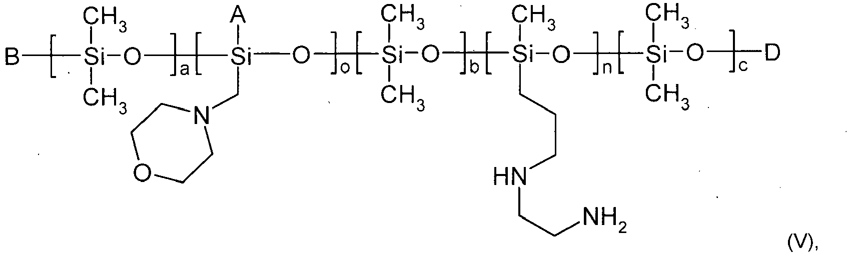 in der
A für eine über ein -O- gebundene Struktureinheit (I), (II), (IIIa), (IIIb), (IIIe) oder (IIIf), 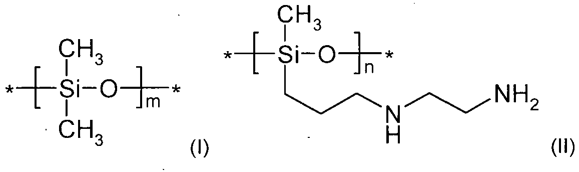 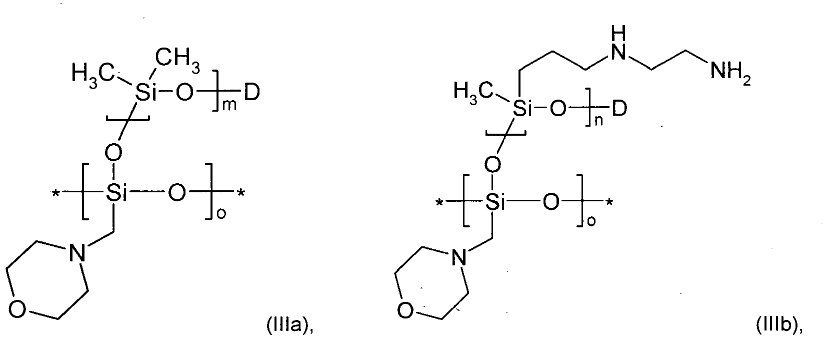 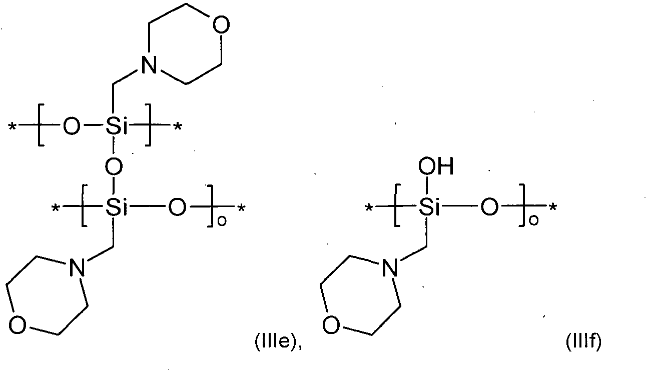 oder für einen über ein -O- gebundenen oligomeren oder polymeren Rest, enthaltend Struktureinheiten der Formeln (I), (II), (IIIa), (IIIb), (IIIe) oder (IIIf) oder für -OH steht,
* für eine Bindung zu einer der Struktureinheiten (I), (II), (IIIa), (IIIb), (IIIe) oder (IIIf) oder für eine Endgruppe B (Si-gebunden) oder D (O-gebunden) steht,
B für eine Gruppe -OH, -O-Si(CH₃)₃, -O-Si(CH₃)₂OH, -O-Si(CH₃)₂OCH₃ steht,
D für eine Gruppe -H, -Si(CH₃)₃, -Si(CH₃)₂OH, -Si(CH₃)₂OCH₃ steht,
a, b und c für ganze Zahlen von 0 bis 990 stehen, mit der Maßgabe a + b + c > 0
m, n und o für ganze Zahlen von 1 bis 990 stehen,
auf die keratinischen Fasern, insbesondere die Haare, aufgetragen wird,
b) anschließend innerhalb eines Zeitraumes von einer Sekunde bis 24 Stunden nach Schritt a) ein Färbe- und/oder Aufhellmittel auf die keratinischen Fasern aufgebracht wird, das durch Vermischen einer Zusammensetzung (A), die mindestens ein Alkalisierungsmittel enthält, mit einer Zusammensetzung (B), die in einem kosmetischen Träger mindestens ein Oxidationsmittel enthält, erhalten wird,
**dadurch gekennzeichnet, dass** die Zusammensetzung (B) mindestens ein Acylpyridiniumderivat der Formel (Acylpyr-I), 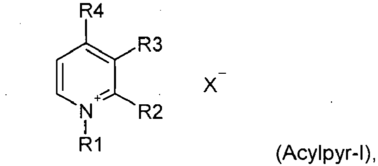 worin
R1 für eine C₁-C₆-Alkylgruppe, eine C₂-C₆-Alkenylgruppe, eine C₂-C₆-Hydroxyalkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
R2, R3 und R4 jeweils unabhängig voneinander für Wasserstoff, eine C₁-C₆-Alkylgruppe, ein Halogenatom oder eine C₁-C₆-Acylgruppe steht, mit der Maßgabe, dass zumindest einer der Reste R2, R3 und R4 für eine C₁-C₆-Acylgruppe steht,
und X⁻ für ein physiologisch verträgliches Anion steht,
enthält.

2. Verfahren nach Anspruch 1, **dadurch gekennzeichnet, dass** das in Schritt a eingesetzte Vorbehandlungsmittel mindestens ein 4-Morpholinomethyl-substituiertes Silikon der Formel (V) enthält, bei dem
m > (n + o) bzw. (a+b+c) > (n + o), bevorzugt
m > 8(n + o) bzw. (ä+b+c) > 8(n + o), besonders bevorzugt
m > 25(n + o) bzw. (a+b+c) > 25(n + o), weiter bevorzugt
m > 77(n + o) bzw. (a+b+c) > 77(n + o) und insbesondere
m > 200(n + o) bzw. (a+b+c) > 200(n + o) gilt.

3. Verfahren nach einem der Ansprüche 1 oder 2, **dadurch gekennzeichnet, dass** das in Schritt a eingesetzte Vorbehandlungsmittel, bezogen auf sein Gewicht, 4-Morpholinomethyl-substituierte(s) Silikon(e) in einer Gesamtmenge von 0,001 bis 5 Gew.-%, bevorzugt 0,005 bis 2 Gew.-%, besonders bevorzugt 0,01 - 1 Gew.-%, außerordentlich bevorzugt 0,02 - 0,1 Gew.-%, jeweils bezogen auf das Gesamtgewicht des Vorbehandlungsmittels, enthält.

4. Verfahren nach einem der Ansprüche 1 bis 3, **dadurch gekennzeichnet, dass** das in Schritt a eingesetzte Vorbehandlungsmittel Hydroxy-terminierte(s) 4-Morpholinomethyl-substituierte(s) Silikon(e) enthält, in denen das Molverhältnis Hydroxy/Alkoxy im Bereich von 0,2:1 bis 0,4:1, bevorzugt im Bereich von 1:0,8 bis 1:1,1 liegt.

5. Verfahren nach einem der Ansprüche 1 bis 4, **dadurch gekennzeichnet, dass** die gewichtsmittlere Molmasse des in Schritt a eingesetzten 4-Morpholinomethyl-substituierten Silikons der Formel (V) im Bereich von 2.000 bis 1.000.000 gmol⁻¹, bevorzugt im Bereich von 5.000 bis 200.000 gmol⁻¹ liegt.

6. Verfahren nach einem der Ansprüche 1 bis 5, **dadurch gekennzeichnet, dass** das in Schritt a eingesetzte 4-Morpholinomethyl-substituierte Silikon der Formel (V) in Form einer Öl-in-Wasser-Emulsion vorliegt, in der die zahlenmittlere Größe der Silicon partikel in der Emulsion bevorzugt im Bereich von 3 bis 500 nm, besonders bevorzugt im Bereich von 5 bis 60 nm liegt.

7. Verfahren nach einem der Ansprüche 1 bis 6, **dadurch gekennzeichnet, dass** als Acylpyridiniumderivat der Formel (Acylpyr-I) mindestens eine Verbindung enthalten ist, bei der entweder R2 oder R4 für eine C₁-C₆-Acylgruppe, bevorzugt für eine Acetylgruppe, steht.

8. Verfahren nach einem der Ansprüche 1 bis 7, **dadurch gekennzeichnet, dass** als Acylpyridiniumderivat der Formel (Acylpyr-I) mindestens eine Verbindung enthalten ist, die ausgewählt ist aus der Gruppe, die gebildet wird aus 4-Acetyl-1-niethylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat, insbesondere 4-Acetyl-1-methylpyridinium-p-toluolsulfonat.

9. Verfahren nach einem der Ansprüche 1 bis 8, **dadurch gekennzeichnet, dass** ein oder mehrere Acylpyridiniumderivat(e) der Formel (Acylpyr-I) in einer Gesamtmenge von 0,001 bis 15 Gew.-%, bevorzugt von 0,01 bis 10 Gew.-% und besonders bevorzugt von 0,1 bis 5 Gew.-%, jeweils bezogen auf das Gesamtgewicht des in Verfahrensschritt b) applizierten Färbe- und/oder Aufhellmittels, enthalten sind.

10. Verfahren nach einem der Ansprüche 1 bis 9, **dadurch gekennzeichnet, dass** man das in Verfahrensschritt a applizierte Vorbehandlungsmittel für einen Zeitraum von 2 Sekunden bis 120 Minuten, bevorzugt 5 Sekunden bis 10 Minuten, auf das Haar einwirken lässt, bevor mindestens einer der folgenden Verfahrensschritte a)i), die vor dem Verfahrensschritt b) erfolgen, erfolgt:
Ausspülen des Haars;
Trocknen des Haars mit einem Handtuch,
Trocknen lassen des Haars an der Luft;
Trockenfönen des Haars,
Trocknen des Haars mit einer Trockenhaube,
Kombinationen der vorgenannten Verfahrensschritte,
wobei der Trockenvorgang bevorzugt bei einer Temperatur von 20°C bis 150°C erfolgt.

11. Verfahren nach Anspruch 10, **dadurch gekennzeichnet, dass** zwischen der in Verfahrensschritt a erfolgten Applikation des Vorbehandlungsmittels und dem Trockenvorgang bzw. den Trockenvorgängen kein Ausspülen des Haars erfolgt.

12. Verfahren nach einem der Ansprüche 1 bis 11, **dadurch gekennzeichnet, dass** die Zusammensetzung (B) mindestens ein kosmetisches Öl in einer Gesamtmenge von 10 - 80 Gew.-%, bevorzugt 2 - 70 Gew.-%, weiter bevorzugt 14 - 60 Gew.-%, besonders bevorzugt 15 - 52 Gew.-% und außerordentlich bevorzugt 17 - 35 Gew.-%, enthält, jeweils bezogen auf das Gewicht der Zusammensetzung (B).

13. Verfahren nach einem der Ansprüche 1 bis 12, **dadurch gekennzeichnet, dass** in Verfahrensschritt b) ein Färbemittel auf die keratinischen Fasern aufgebracht wird, das durch Vermischen einer Zusammensetzung (A), die mindestens ein Alkalisierungsmittel, mindestens ein Oxidationsfarbstoffvorprodukt vom Entwicklertyp und mindestens ein Oxidationsfarbstoffvorprodukt vom Kupplertyp enthält, mit der Zusammensetzung (B) erhalten wird.

14. Mehrkomponentenverpackungseinheit (Kit-of-Parts), umfassend
einen ersten Container (C1) mit einem Mittel, enthaltend mindestens ein 4-Morpholinomethyl-substituiertes Silikon der Formel (V), 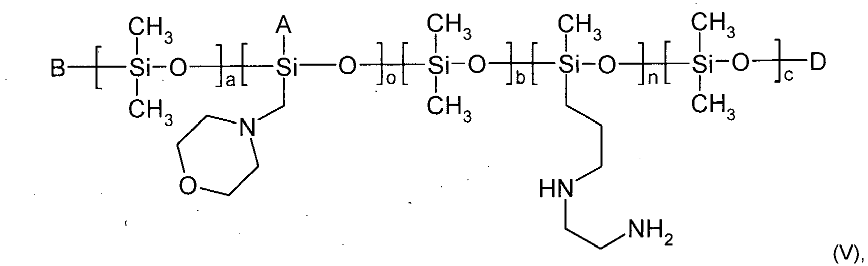 in der
A für eine über ein -O- gebundene Struktureinheit (I), (II), (IIIa), (IIIb), (IIIe) oder (IIIf), 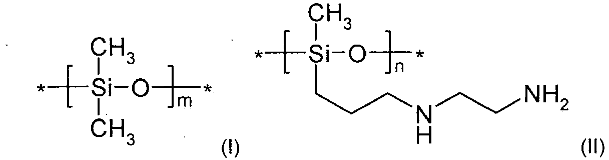 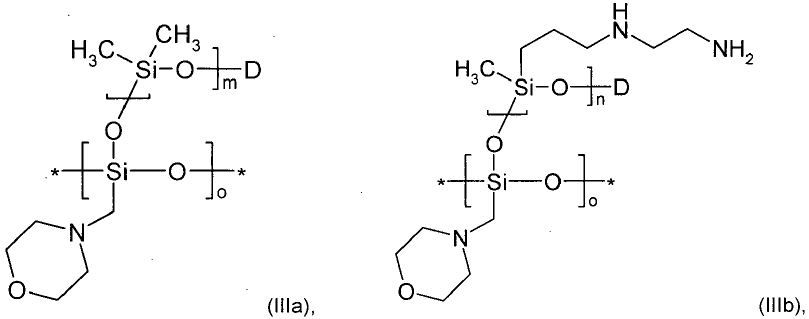 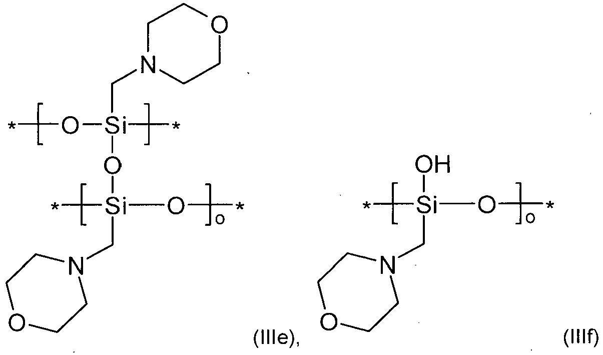 oder für einen über ein -O- gebundenen oligomeren oder polymeren Rest, enthaltend Struktureinheiten der Formeln (I), (II), (IIIa), (IIIb), (IIIe) oder (IIIf) oder für -OH steht,
* für eine Bindung zu einer der Struktureinheiten (I), (II), (IIIa), (IIIb), (IIIe) oder (IIIf) oder für eine Endgruppe B (Si-gebunden) oder D (O-gebunden) steht,
B für eine Gruppe -OH, -O-Si(CH₃)₃, -O-Si(CH₃)₂OH, -O-Si(CH₃)₂OCH₃ steht,
D für eine Gruppe -H, -Si(CH₃)₃, -Si(CH₃)₂OH, -Si(CH₃)₂OCH₃ steht,
a, b und c für ganze Zahlen von 0 bis 990 stehen, mit der Maßgabe a + b + c > 0
m, n und o für ganze Zahlen von 1 bis 990 stehen,
weiterhin einen zweiten Container (C2) mit einer Zusammensetzung (A), enthaltend mindestens ein Alkalisierungsmittel,
und weiterhin einen dritten Container (C3) mit einer Zusammensetzung (B), enthaltend in einem kosmetischen Träger mindestens ein Oxidationsmittel und mindestens ein Acylpyridiniumderivat der Formel (Acylpyr-I) 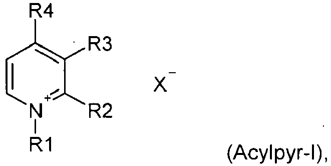 worin
R1 für eine C₁-C₆-Alkylgruppe, eine C₂-C₆-Alkenylgruppe, eine C₂-C₆-Hydroxyalkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
R2, R3 und R4 jeweils unabhängig voneinander für Wasserstoff, eine C₁-C₆-Alkylgruppe, ein Halogenatom oder eine C₁-C₆-Acylgruppe steht, mit der Maßgabe, dass zumindest einer der Reste R2, R3 und R4 für eine C₁-C₆-Acylgruppe steht,
und X⁻ für ein physiologisch verträgliches Anion steht.

## Claims

1. A method for oxidatively brightening and/or dyeing keratin fibers, in particular hair, **characterized in that**
a) a pre-treatment agent that contains at least one 4-morpholinomethyl-substituted silicone of formula (V), 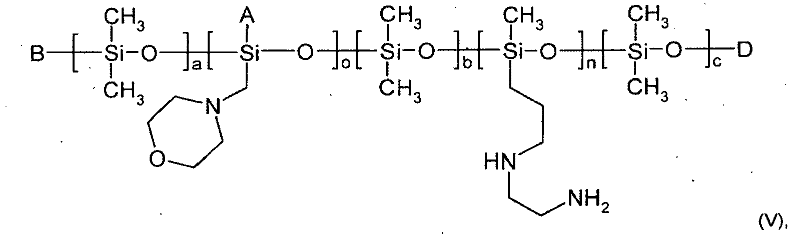 in which
A represents a structural unit (I), (II), (IIIa), (IIIb), (IIIe) or (IIIf) bound by an-O-, 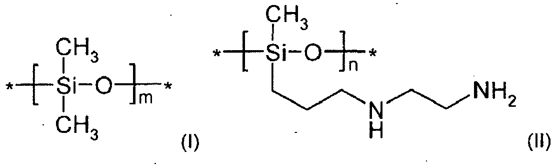 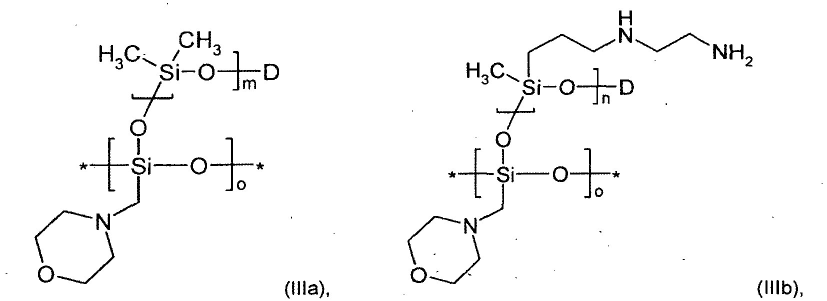 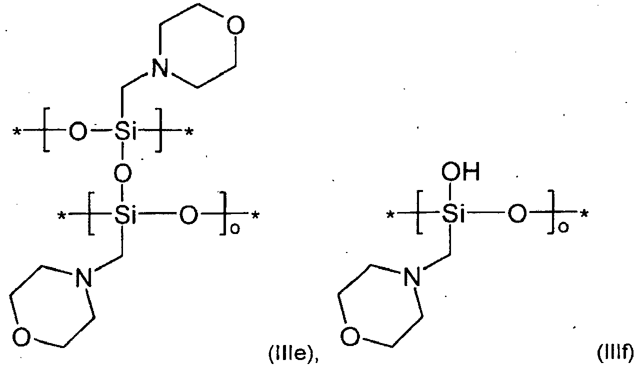 or an oligomeric or polymeric functional group bound by an -O-, containing structural units of formulae (I), (II), (IIIa), (IIIb), (IIIe) or (IIIf), or -OH,
* represents a bond to one of the structural units (I), (II), (IIIa), (IIIb), (IIIe) or (IIIf), or a terminal group B (Si-bound) or D (O-bound),
B represents an -OH, -O-Si(CH₃)₃, -O-Si(CH₃)₂OH or -O-Si(CH₃)₂OCH₃ group,
D represents a -H, -Si(CH₃)₃, -Si(CH₃)₂OH, -Si(CH₃)₂OCH₃ group,
a, b and c represent integers from 0 to 990, with the proviso a + b + c > 0
m, n and o represent integers from 1 to 990,
is applied to the keratin fibers, in particular the hair,
b) subsequently, within a period of from one second to 24 hours, after step a) a dye and/or brightening agent is applied to the keratin fibers, which is obtained by mixing a composition (A), which contains at least one alkalizing agent, with a composition (B), which contains at least one oxidizing agent in a cosmetic carrier,
**characterized in that** the composition (B) contains at least one acyl pyridinium derivative of formula (Acylpyr-I), 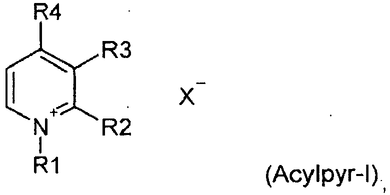 in which
R1 represents a C₁-C₆ alkyl group, a C₂-C₆ alkenyl group, a C₂-C₆ hydroxyalkyl group, a C₁-C₆ alkoxy C₂-C₆ alkyl group, a carboxy C₂-C₆ alkyl group, an aryl C₁-C₆ alkyl group, a heteroaryl C₁-C₆ alkyl group, an aryl group or a heteroaryl group,
R2, R3 and R4 each represent, independently of one another, hydrogen, a C₁-C₆ alkyl group, a halogen atom or a C₁-C₆ acyl group, with the proviso that at least one of the functional groups R2, R3 and R4 represents a C₁-C₆ acyl group,
and X⁻ represents a physiologically acceptable anion.

2. The method according to claim 1, **characterized in that** the pre-treatment agent used in step a) contains at least one 4-morpholinomethyl-substituted silicone of formula (V), in which
m > (n + o) or (a+b+c) > (n + o), preferably
m > 8(n + o) or (a+b+c) > 8(n + o), particularly preferably
m > 25(n + o) or (a+b+c) > 25(n + o), more preferably
m > 77(n + o) or (a+b+c) > 77(n + o) and in particular
m > 200(n + o) or (a+b+c) > 200(n + o) applies.

3. The method according to one of claims 1 or 2, **characterized in that** the pre-treatment agent used in step a), based on its weight, contains 4-morpholinomethyl-substituted silicone(s) in a total amount of from 0.001 to 5 wt.%, preferably from 0.005 to 2 wt.%, particularly preferably from 0.01 to 1 wt.%, exceptionally preferably from 0.02 to 0.1 wt.%, in each case based on the total weight of the pre-treatment agent.

4. The method according to one of claims 1 to 3, **characterized in that** the pre-treatment agent used in step a) contains hydroxy-terminated 4-morpholinomethyl-substituted silicone(s), in which the molar ratio hydroxy/alkoxy is in the range of from 0.2:1 to 0.4:1, preferably in the range of from 1:0.8 to 1:1.1.

5. The method according to one of claims 1 to 4, **characterized in that** the weight-average molar mass of the 4-morpholinomethyl-substituted silicone of formula (V) used in step a) is in the range of from 2,000 to 1,000,000 gmol⁻¹, preferably in the range of from 5,000 to 200,000 gmol⁻¹.

6. The method according to one of claims 1 to 5, **characterized in that** the 4-morpholinomethyl-substituted silicone of formula (V) used in step a) is in the form of an oil-in-water emulsion, in which the number-average size of the silicone particles in the emulsion is preferably in the range of from 3 to 500 nm, particularly preferably in the range of from 5 to 60 nm.

7. The method according to one of claims 1 to 6, **characterized in that**, as the acyl pyridinium derivative of formula (Acylpyr-I), at least one compound is contained in which either R2 or R4 represents a C₁-C₆ acyl group, preferably an acetyl group.

8. The method according to one of claims 1 to 7, **characterized in that**, as the acyl pyridinium derivative of formula (Acylpyr-I), at least one compound is contained that is selected from the group that is formed by 4-acetyl-1-methylpyridinium p-toluenesulfonate, 4-acetyl-1-methylpyridinium benzenesulfonate, 4-acetyl-1-methylpyridinium hydrogen sulfate, 4-acetyl-1-methylpyridinium acetate, 4-acetyl-1-allylpyridinium p-toluenesulfonate, 4-acetyl-1-allylpyridinium benzenesulfonate, 4-acetyl-1-allylpyridinium hydrogen sulfate, 4-acetyl-1-allylpyridinium acetate, 2-acetyl-1-methylpyridinium p-toluenesulfonate, 2-acetyl-1-methylpyridinium benzenesulfonate, 2-acetyl-1-methylpyridinium hydrogen sulfate, 2-acetyl-1-methylpyridinium acetate, 2-acetyl-1-allylpyridinium p-toluenesulfonate, 2-acetyl-1-allylpyridinium benzenesulfonat, 2-acetyl-1-allylpyridinium hydrogen sulfate, and 2-acetyl-1-allylpyridinium acetate, in particular 4-acetyl-1-methylpyridinium p-toluenelsulfonate.

9. The method according to one of claims 1 to 8, **characterized in that** one or more acyl pyridinium derivatives of formula (Acylpyr-I) are contained in a total amount of from 0.001 to 15 wt.%, preferably from 0.01 to 10 wt.%, and particularly preferably from 0.1 to 5 wt.%, in each case based on the total weight of the dye and/or brightening agent applied in method step b).

10. The method according to one of claims 1 to 9, **characterized in that** the pre-treatment agent applied in method step a) is allowed to act on the hair for a period of from 2 seconds to 120 minutes, preferably from 5 seconds to 10 minutes, before at least one of the following method steps a)i), which take place before method step (b):
rinsing the hair;
drying the hair using a towel;
allowing the hair to air-dry;
blow-drying the hair;
drying the hair using a dryer hood;
combinations of the above-mentioned method steps,
the drying process preferably taking place at a temperature of from 20 °C to 150 °C.

11. The method according to claim 10, **characterized in that**, between the application of the pre-treatment agent in method step a) and the drying process or the drying processes, the hair is not rinsed.

12. The method according to one of claims 1 to 11, **characterized in that** the composition (B) contains at least one cosmetic oil in a total amount of from 10 to 80 wt.%, preferably from 2 to 70 wt.%, more preferably from 14 to 60 wt.%, particularly preferably from 15 to 52 wt.%, and exceptionally preferably from 17 to 35 wt.%, in each case based on the weight of the composition (B).

13. The method according to one of claims 1 to 12, **characterized in that**, in method step b), a dye is applied to the keratin fibers, which is obtained by mixing a composition (A), which contains at least one alkalizing agent, at least one developer-type oxidation dye precursor, and at least one coupler-type oxidation dye precursor, with the composition (B).

14. A kit of parts, comprising
a first container (C1) comprising an agent, containing at least one 4-morpholinomethyl-substituted silicone of formula (V), 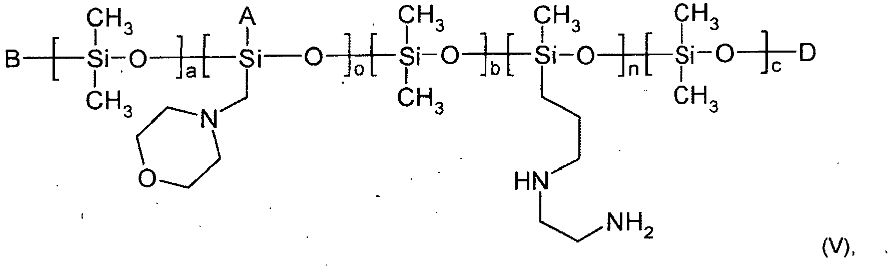 in which
A represents a structural unit (I), (II), (IIIa), (IIIb), (IIIe) or (IIIf) bound by an-O-, 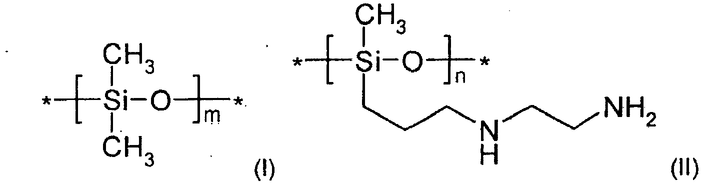 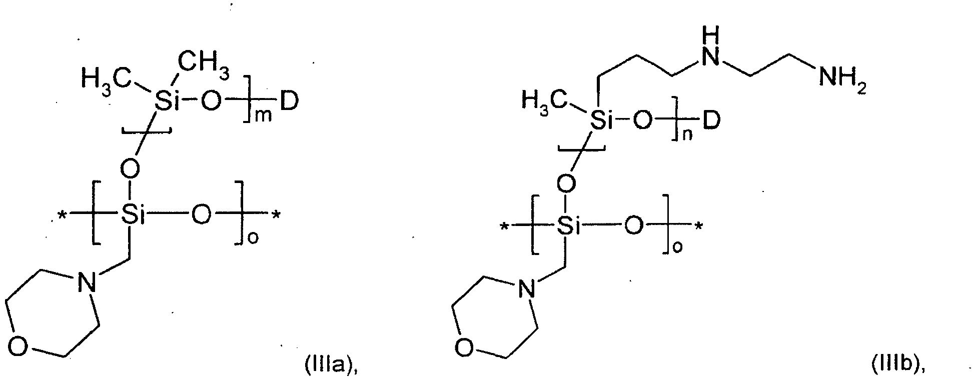 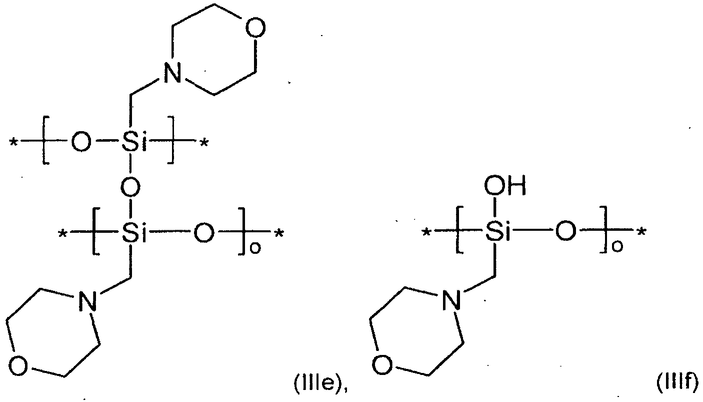 or an oligomeric or polymeric functional group bound by an -O-, containing structural units of formulae (I), (II), (IIIa), (IIIb), (IIIe) or (IIIf), or -OH,
* represents a bond to one of the structural units (I), (II), (IIIa), (IIIb), (IIIe) or (IIIf), or a terminal group B (Si-bound) or D (O-bound),
B represents an -OH, -O-Si(CH₃)₃, -O-Si(CH₃)₂OH or -O-Si(CH₃)₂OCH₃ group,
D represents a -H, -Si(CH₃)₃, -Si(CH₃)₂OH, -Si(CH₃)₂OCH₃ group,
a, b and c represent integers from 0 to 990, with the proviso a + b + c > 0
m, n and o represent integers from 1 to 990,
also
a second container (C2) comprising a composition (A), containing at least one alkalizing agent,
and also a third container (C3) comprising a composition (B), containing in a cosmetic carrier at least one oxidizing agent and at least one acyl pyridinium derivative of formula (Acylpyr-I) 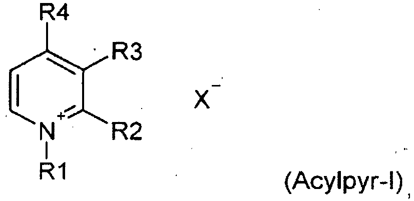 in which
R1 represents a C₁-C₆ alkyl group, a C₂-C₆ alkenyl group, a C₂-C₆ hydroxyalkyl group, a C₁-C₆ alkoxy C₂-C₆ alkyl group, a carboxy C₂-C₆ alkyl group, an aryl C₁-C₆ alkyl group, a heteroaryl C₁-C₆ alkyl group, an aryl group or a heteroaryl group,
R2, R3 and R4 each represent, independently of one another, hydrogen, a C₁-C₆ alkyl group, a halogen atom or a C₁-C₆ acyl group, with the proviso that at least one of the functional groups R2, R3 and R4 represents a C₁-C₆ acyl group,
and X⁻ represents a physiologically acceptable anion.

## Revendications

1. Procédé d'éclaircissement et/ou de coloration par oxydation de fibres kératiniques, notamment de cheveux humains, **caractérisé en ce que**
a) un agent de prétraitement qui contient au moins une silicone substituée 4-morpholinométhyle de la formule (V) 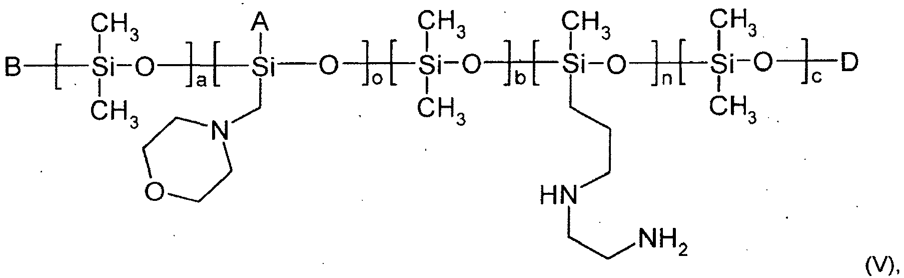 dans laquelle
A représente une unité structurelle (I), (II), (IIIa), (IIIb), (IIIe) ou (IIIf) liée par un -O-, 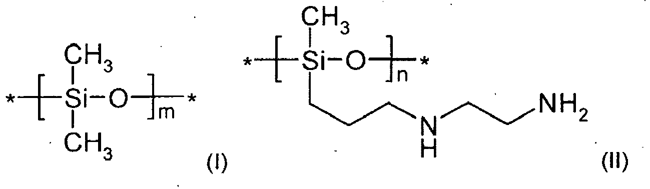 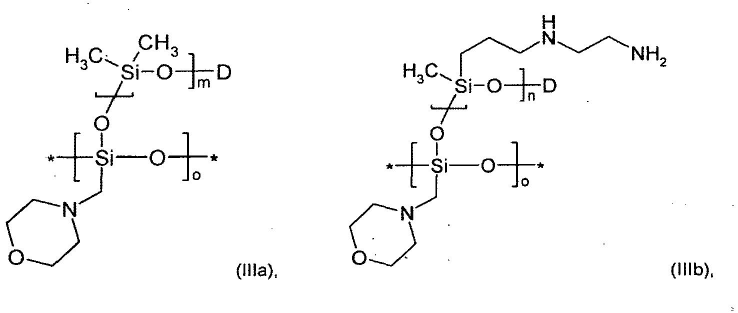 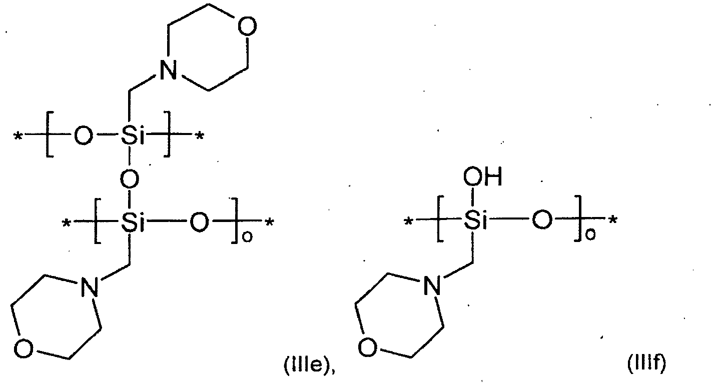 ou un résidu oligomère ou polymère lié par un -O-, contenant des unités structurelles des formules (I), (II), (IIIa), (IIIb), (IIIe) ou (IIIf), ou -OH
* représente une fixation vers une des unités structurelles (I), (II), (IIIa), (IIIb), (IIIe) ou (IIIf) ou représente un groupe d'extrémité B (lié Si) ou D (lié O),
B représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃,
a, b et c représentent des nombres entiers de 0 à 990, à la condition que a + b + c > 0
m, n et o représentent des nombres entiers de 1 à 990,
est appliqué sur les fibres kératiniques, notamment des cheveux humains,
b) puis un agent colorant et/ou éclaircissant est appliqué sur les fibres kératiniques dans un intervalle de temps d'une seconde à 24 heures après l'étape a), qui est obtenu par mélange d'une composition (A), qui contient au moins un agent d'alcalinisation, avec une composition (B), qui contient au moins un agent d'oxydation dans un support cosmétique,
**caractérisé en ce que** la composition (B) contienne au moins un dérivé acylpyridinium de la formule (acylpyr-1), 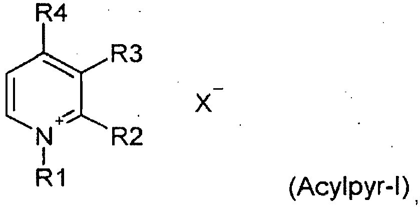 dans laquelle
R1 représente un groupe alkyle en C₁-C₆, un groupe alcényle en C₂-C₆, un groupe hydroxyalkyle en C₂-C₆, un groupe alkyle en C₂-C₆ alcoxy en C₁-C₆, un groupe carboxyalkyle en C₂-C₆, un groupe arylalkyle en C₁-C₆, un groupe hétéroarylalkyle en C₁-C₆, un groupe aryle ou un groupe hétéroaryle,
R2, R3 et R4 représentent respectivement indépendamment l'un de l'autre de l'hydrogène, un groupe alkyle en C₁-C₆, un atome d'halogène ou un groupe acyle en C₁-C₆, à la condition qu'au moins un des résidus R2, R3 et R4 représente un groupe acyle en C₁-C₆,
et X⁻ représente un anion physiologiquement compatible.

2. Procédé selon la revendication 1, **caractérisé en ce que** l'agent de prétraitement utilisé dans l'étape a) contient au moins une silicone substituée 4-morpholinométhyle de la formule (V), dans lequel on respecte les paramètres suivants
m > (n + o), respectivement (a+b+c) > (n + o), préférablement
m > 8(n + o) respectivement (a+b+c) > 8(n + o), très préférablement
m > 25(n + o) respectivement (a+b+c) > 25(n + o), plus préférablement
m > 77(n + o) respectivement (a+b+c) > 77(n + o) et en particulier
m > 200(n + o) respectivement (a+b+c) > 200(n + o).

3. Procédé selon l'une quelconque des revendications 1 ou 2, **caractérisé en ce que** l'agent de prétraitement utilisé dans l'étape a) contient, rapporté à son poids, une/des silicone(s) substituée(s) 4-morpholinométhyle dans une quantité totale allant de 0,001 à 5 % en poids, préférablement 0,005 à 2 % en poids, très préférablement 0,01 - 1 % en poids, on ne peut plus préférablement 0,02 - 0,1 % en poids, respectivement rapporté au poids total de l'agent de prétraitement.

4. Procédé selon l'une quelconque des revendications 1 à 3, **caractérisé en ce que** l'agent de prétraitement utilisé dans l'étape a) contient une/des silicone(s) substituée(s) 4-morpholinométhyle à terminaison hydroxy, dans lesquelles le rapport molaire hydroxy/alcoxy est compris dans une plage allant de 0,2:1 à 0,4:1, préférablement dans une plage allant de 1:0,8 à 1:1,1.

5. Procédé selon l'une quelconque des revendications 1 à 4, **caractérisé en ce que** la masse molaire moyenne en poids de la silicone substituée 4-morpholinométhyle de la formule (V), utilisée dans l'étape a), est comprise dans une plage allant de 2 000 à 1 000 000 gmol⁻¹, préférablement dans une plage allant de 5 000 à 200 000 gmol⁻¹.

6. Procédé selon l'une quelconque des revendications 1 à 5, **caractérisé en ce que** la silicone substituée 4-morpholinométhyle de la formule (V), utilisée dans l'étape a), se présente sous la forme d'une émulsion huile-dans-l'eau, dans laquelle la taille moyenne des particules de silicone dans l'émulsion est comprise préférablement dans une plage allant de 3 à 500 nm, très préférablement dans une plage allant de 5 à 60 nm.

7. Procédé selon l'une quelconque des revendications 1 à 6, **caractérisé en ce qu'**au moins un composé est présent en tant que dérivé d'acylpyridinium de la formule (acylpyr-I), dans laquelle soit R2, soit R4 représente un groupe acyle en C₁-C₆, préférablement représente un groupe acétyle.

8. Procédé selon l'une quelconque des revendications 1 à 7, **caractérisé en ce qu'**au moins un composé est présent en tant que dérivé d'acylpyridinium de la formule (acylpyr-I), sélectionné parmi le groupe constitué du p-toluolsulfonate de 4-acétyl-1-méthylpyridinium, du benzolsulfonate de 4-acétyl-1-méthylpyridinium, de l'hydrogénosulfate de 4-acétyl-1-méthylpyridinium, de l'acétate de 4-acétyl-1-méthylpyridinium, du p-toluolsulfonate de 4-acétyl-1-allylpyridinium, du benzolsulfonate de 4-acétyl-1-allylpyridinium, de l'hydrogénosulfate de 4-acétyl-1-allylpyridinium, de l'acétate de 4-acétyl-1-allylpyridinium, du p-toluolsulfonate de 2-acétyl-1-méthylpyridinium, du benzolsulfonate de 2-acétyl-1-méthylpyridinium, de l'hydrogénosulfate de 2-acétyl-1-méthylpyridinium, de l'acétate de 2-acétyl-1-méthylpyridinium, du p-toluolsulfonate de 2-acétyl-1-allylpyridinium, du benzolsulfonate de 2-acétyl-1-allylpyridinium, de l'hydrogénosulfonate de 2-acétyl-1-allylpyridinium et de l'acétate de 2-acétyl-1-allylpyridinium, notamment du p-toluolsulfonate de 4-acétyl-1-méthylpyridinium.

9. Procédé selon l'une quelconque des revendications 1 à 8, **caractérisé en ce qu'**un ou plusieurs dérivé(s) d'acylpyridinium de la formule (acylpyr-I) sont présents dans une quantité totale de 0,001 à 15 % en poids, préférablement de 0,01 à 10 % en poids et très préférablement de 0,1 à 5 % en poids, respectivement rapporté au poids total de l'agent colorant et/ou éclaircissant appliqué dans l'étape b) du procédé.

10. Procédé selon l'une quelconque des revendications 1 à 9, **caractérisé en ce que** l'on laisse agir sur le cheveu l'agent de prétraitement appliqué dans l'étape a) du procédé pour une durée de 2 secondes à 120 minutes, préférablement 5 secondes à 10 minutes, avant que ne se produise au moins une des étapes a)i) du procédé, qui se produisent avant l'étape b) du procédé :
rinçage du cheveu ;
séchage du cheveu avec une serviette de bain,
séchage passif du cheveu à l'air libre ;
séchage du cheveu au sèche-cheveux,
séchage du cheveu à l'aide d'un séchoir,
combinaisons des étapes de procédé précédemment mentionnées,
dans lequel le procédé de séchage s'effectue préférablement à une température de 20 °C à 150 °C.

11. Procédé selon la revendication 10, **caractérisé en ce qu'**entre l'application de l'agent de prétraitement effectuée à l'étape a) du procédé et le procédé de séchage ou des procédés de séchage, il n'y a pas de rinçage du cheveu.

12. Procédé selon l'une quelconque des revendications 1 à 11, **caractérisé en ce que** la composition (B) contient au moins une huile cosmétique dans une quantité totale allant de 10 - 80 % en poids, préférablement 2 - 70 % en poids, plus préférablement 14 - 60 % en poids, très préférablement 15 - 52 % en poids et on ne peut plus préférablement 17 - 35 % en poids, respectivement rapporté au poids de la composition (B).

13. Procédé selon l'une quelconque des revendications 1 à 12, **caractérisé en ce qu'**un agent colorant est appliqué sur les fibres kératiniques lors de l'étape b) du procédé, lequel est obtenu par mélange d'une composition (A), qui contient au moins un agent d'alcalinisation, au moins un précurseur de colorant par oxydation du type développeur et au moins un précurseur de colorant par oxydation du type coupleur, avec la composition (B).

14. Unité de conditionnement à composants multiples (kit-of-parts), comprenant un premier conteneur (C1) avec un agent, contenant au moins une silicone substituée 4-morpholinométhyle de la formule (V), 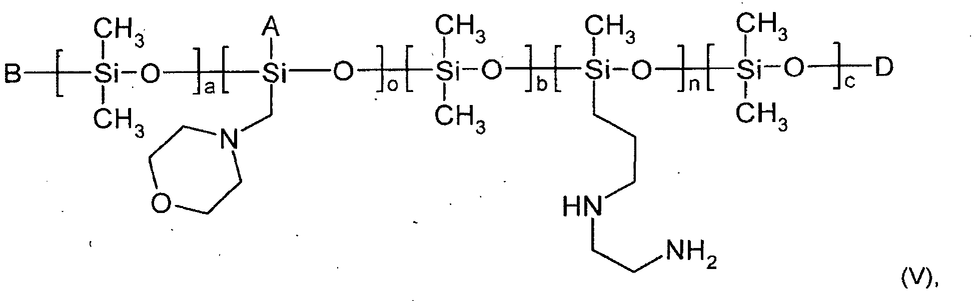 dans laquelle
A représente une unité structurelle (I), (II), (IIIa), (IIIb), (IIIe) ou (IIIf) liée par un -O-, 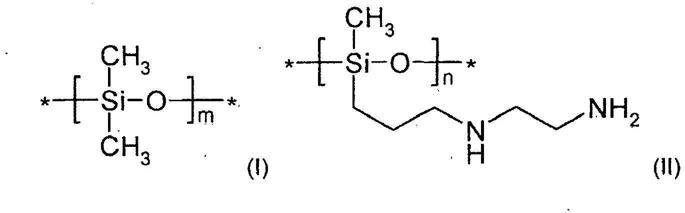 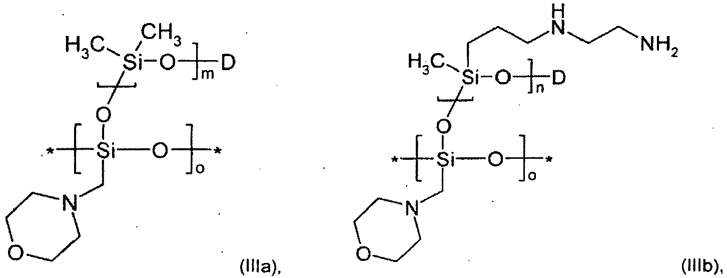 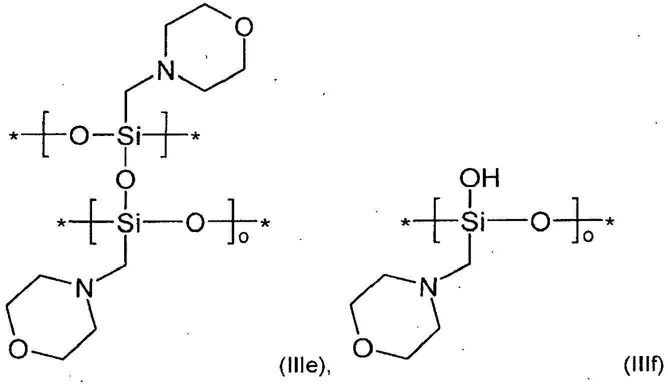 ou représente un résidu oligomère ou polymère lié par un -O-, contenant des unités structurelles des formules (I), (II), (IIIa), (IIIb), (IIIe) ou (IIIf), ou -OH
* représente une fixation vers une des unités structurelles (I), (II), (IIIa), (IIIb), (IIIe) ou (IIIf) ou représente un groupe d'extrémité B (lié Si) ou D (lié O),
B représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃,
a, b et c représentent des nombres entiers de 0 à 990, à la condition que a + b + c > 0
m, n et o représentent des nombres entiers de 1 à 990,
en outre
un deuxième conteneur (C2) avec une composition (A), contenant au moins un agent d'alcalinisation,
et en outre un troisième conteneur (C3) avec une composition (B), contenant dans un support cosmétique au moins un agent d'oxydation et au moins un dérivé acylpyridinium de la formule (acylpyr-I) 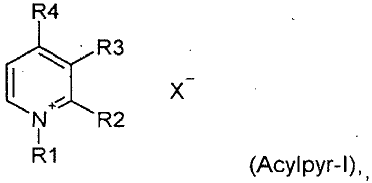 dans laquelle
R1 représente un groupe alkyle en C₁-C₆, un groupe alcényle en C₂-C₆, un groupe hydroxyalkyle en C₂-C₆, un groupe alkyle en C₂-C₆ alcoxy en C₁-C₆, un groupe carboxyalkyle en C₂-C₆, un groupe arylalkyle en C₁-C₆, un groupe hétéroarylalkyle en C₁-C₆, un groupe aryle ou un groupe hétéroaryle,
R2, R3 et R4 représentent respectivement indépendamment l'un de l'autre de l'hydrogène, un groupe alkyle en C₁-C₆, un atome d'halogène ou un groupe acyle en C₁-C₆, à la condition qu'au moins un des résidus R2, R3 et R4 représente un groupe acyle en C₁-C₆,
et X⁻ représente un anion physiologiquement compatible.
